(19) Europäisches Patentamt / European Patent Office / Office européen des brevets

(11) **EP 2 632 938 B1**

(12) **FASCICULE DE BREVET EUROPEEN**

(45) Date de publication et mention
de la délivrance du brevet:
**26.02.2020 Bulletin 2020/09**

(21) Numéro de dépôt: **11785744.1**

(22) Date de dépôt: **24.10.2011**

(51) Int Cl.:
*C07K 5/10* *(2006.01)*     *C07K 5/117* *(2006.01)*
*C07K 5/113* *(2006.01)*     *C07K 5/11* *(2006.01)*
*A61K 38/07* *(2006.01)*     *A61P 25/28* *(2006.01)*

(86) Numéro de dépôt international:
**PCT/FR2011/052477**

(87) Numéro de publication internationale:
**WO 2012/056157 (03.05.2012 Gazette 2012/18)**

(54) **COMPOSE PEPTIDIQUE UTILE POUR L'INHIBITION DE LA FORMATION DE PLAQUES AMYLOÏDES**

PEPTIDVERBINDUNG ZUR HEMMUNG EINER AMYLOIDPLAQUEBILDUNG

PEPTIDE COMPOUND USEFUL FOR INHIBITING AMYLOID PLAQUE FORMATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.10.2010 FR 1058827**

(43) Date de publication de la demande:
**04.09.2013 Bulletin 2013/36**

(73) Titulaire: **Kimonella Ventures Ltd**
**1060 Nicosie (CY)**

(72) Inventeurs:
• **MEDIANNIKOV, Oleg**
**13004 Marseille (FR)**
• **MOROZOV, Alexander**
**111398 Moscou (RU)**

(74) Mandataire: **Cabinet Beau de Loménie**
**Tour Méditerranée**
**65 avenue Jules Cantini**
**13006 Marseille (FR)**

(56) Documents cités:
**EP-A1- 2 130 550     WO-A1-93/13789**
**WO-A2-01/96364     US-A1- 2004 214 165**

• **CHU Y -H ET AL: "Free solution identification of candidate peptides from combinatorial libraries by affinity capillary electrophoresis/mass spectrometry", JOURNAL OF THE AMERICAN CHEMICAL SOCIETY 19950517 US, vol. 117, no. 19, 17 mai 1995 (1995-05-17) , pages 5419-5420, XP002632308, ISSN: 0002-7863**
• **GOMEZ-RUIZ J A ET AL: "Sensory and Mass Spectrometric Analysis of the Peptidic Fraction Lower Than One Thousand Daltons in Manchego Cheese", JOURNAL OF DAIRY SCIENCE, AMERICAN DAIRY SCIENCE ASSOCIATION, US, vol. 90, no. 11, 1 November 2007 (2007-11-01), pages 4966-4973, XP026956245, ISSN: 0022-0302 [retrieved on 2007-11-01]**

SEQUENCE LISTING

<110> KIMONELLA VENTURES LTD

<120> COMPOSE PEPTIDIQUE UTILE POUR L'INHIBITION DE LA FORMATION DE PLAQUES AMYLODES

<130> 3H53 240 cas 1 PCT

<150> FR 10/58827
<151> 2010-10-27

<160> 68

<170> PatentIn version 3.5

<210> 1
<211> 4
<212> PRT
<213> artificial sequence

<220>

<223> Tétrapeptide interagissant avec la région 11-14 de l'amyloïde béta

<400> 1

```
                                His Ala Asp Asp
                                1
```

<210> 2
<211> 4
<212> PRT
<213> artificial sequence

<220>

<223> Tétrapeptide interagissant avec la région 11-14 de l'amyloïde béta

<400> 2

```
                                Asp Asp Ala His
                                1
```

<210> 3
<211> 4
<212> PRT
<213> artificial sequence

<220>

<223> Tétrapeptide interagissant avec la région 11-14 de l'amyloïde béta

<400> 3

```
                                Lys Ala Asp Asp
                                1
```

<210> 4
<211> 4
<212> PRT
<213> artificial sequence

<220>

<223> Tétrapeptide interagissant avec la région 11-14 de l'amyloïde béta

<400> 4

```
                                Asp Asp Ala Lys
                                1
```

<210> 5
<211> 4
<212> PRT
<213> artificial sequence

<220>

<223> Tétrapeptide interagissant avec la région 11-14 de l'amyloïde béta

<400> 5

                                        **Arg Ala Asp Asp**
                                        **1**

<210> 6
<211> 4
<212> PRT
<213> artificial sequence

<220>
<223> Tétrapeptide interagissant avec la région 11-14 de l'amyloïde béta

<400> 6

                                        **Asp Asp Ala Arg**
                                        **1**

<210> 7
<211> 4
<212> PRT
<213> artificial sequence

<220>
<223> Tétrapeptide interagissant avec la région 11-14 de l'amyloïde béta

<400> 7

                                        **His Ala Glu Asp**
                                        **1**

<210> 8
<211> 4
<212> PRT
<213> artificial sequence

<220>
<223> Tétrapeptide interagissant avec la région 11-14 de l'amyloïde béta

<400> 8

                                        **Asp Glu Ala His**
                                        **1**

<210> 9
<211> 4
<212> PRT
<213> artificial sequence

<220>
<223> Tétrapeptide interagissant avec la région 11-14 de l'amyloïde béta

<400> 9

                                        **Lys Ala Glu Asp**
                                        **1**

<210> 10
<211> 4
<212> PRT

<213> artificial sequence

<220>
<223> Tétrapeptide interagissant avec la région 11-14 de l'amyloïde béta

<400> 10

Asp Glu Ala Lys
1

<210> 11
<211> 4
<212> PRT
<213> artificial sequence

<220>
<223> Tétrapeptide interagissant avec la région 11-14 de l'amyloïde béta

<400> 11

Arg Ala Glu Asp
1

<210> 12
<211> 4
<212> PRT
<213> artificial sequence

<220>
<223> Tétrapeptide interagissant avec la région 11-14 de l'amyloïde béta

<400> 12

Asp Glu Ala Arg
1

<210> 13
<211> 4
<212> PRT
<213> artificial sequence

<220>
<223> Tétrapeptide interagissant avec la région 11-14 de l'amyloïde béta

<400> 13

His Ala Asp Glu
1

<210> 14
<211> 4
<212> PRT
<213> artificial sequence

<220>
<223> Tétrapeptide interagissant avec la région 11-14 de l'amyloïde béta

<400> 14

```
Glu Asp Ala His
1
```

<210> 15
<211> 4
<212> PRT
<213> artificial sequence

<220>
<223> Tétrapeptide interagissant avec la région 11-14 de l'amyloïde béta

<400> 15

```
Lys Ala Asp Glu
1
```

<210> 16
<211> 4
<212> PRT
<213> artificial sequence

<220>
<223> Tétrapeptide interagissant avec la région 11-14 de l'amyloïde béta

<400> 16

```
Glu Asp Ala Lys
1
```

<210> 17
<211> 4
<212> PRT
<213> artificial sequence

<220>
<223> Tétrapeptide interagissant avec la région 11-14 de l'amyloïde béta

<400> 17

```
Arg Ala Asp Glu
1
```

<210> 18
<211> 4
<212> PRT
<213> artificial sequence

<220>
<223> Tétrapeptide interagissant avec la région 11-14 de l'amyloïde béta

<400> 18

```
Glu Asp Ala Arg
1
```

<210> 19
<211> 4
<212> PRT

<213> artificial sequence

<220>
<223> Tétrapeptide interagissant avec la région 11-14 de l'amyloïde béta

<400> 19

His Ala Glu Glu
1

<210> 20
<211> 4
<212> PRT
<213> artificial sequence

<220>
<223> Tétrapeptide interagissant avec la région 11-14 de l'amyloïde béta

<400> 20

Glu Glu Ala His
1

<210> 21
<211> 4
<212> PRT
<213> artificial sequence

<220>
<223> Tétrapeptide interagissant avec la région 11-14 de l'amyloïde béta

<400> 21

Lys Ala Glu Glu
1

<210> 22
<211> 4
<212> PRT
<213> artificial sequence

<220>
<223> Tétrapeptide interagissant avec la région 11-14 de l'amyloïde béta

<400> 22

Glu Glu Ala Lys
1

<210> 23
<211> 4
<212> PRT
<213> artificial sequence

<220>
<223> Tétrapeptide interagissant avec la région 11-14 de l'amyloïde béta

<400> 23

```
Arg Ala Glu Glu
1
```

<210> 24
<211> 4
<212> PRT
<213> artificial sequence

<220>
<223> Tétrapeptide interagissant avec la région 11-14 de l'amyloïde béta

<400> 24

```
Glu Glu Ala Arg
1
```

<210> 25
<211> 16
<212> PRT
<213> artificial sequence

<220>
<223> Région 1-16 de l'amyloïde béta

<400> 25

```
Asp Ala Glu Phe Arg His Asp Ser Gly Tyr Glu Val His His Gln Lys
1               5               10              15
```

<210> 26
<211> 21
<212> PRT
<213> artificial sequence

<220>
<223> Fragment peptidique synthétique contenant la région 1-16 de l'amyloïde béta

<400> 26

```
Asp Ala Glu Phe Arg His Asp Ser Gly Tyr Glu Val His His Gln Lys
1               5               10              15

Gly Gly Gly Gly Cys
                20
```

<210> 27
<211> 4
<212> PRT
<213> artificial sequence

<220>
<223> Région 13-16 de l'amyloïde béta

<400> 27

```
His His Gln Lys
1
```

<210> 28
<211> 4
<212> PRT
<213> artificial sequence

<220>
<223> Région 11-14 de l'amyloïde béta

<400> 28

Glu Val His His
1

<210> 29
<211> 4
<212> PRT
<213> artificial sequence

<220>
<223> Tétrapeptide selon l'invention modifié par une alanine

<400> 29

Ala Ala Glu Glu
1

<210> 30
<211> 4
<212> PRT
<213> artificial sequence

<220>
<223> Tétrapeptide selon l'invention modifié par une alanine

<400> 30

His Ala Glu Ala
1

<210> 31
<211> 4
<212> PRT
<213> artificial sequence

<220>
<223> Tétrapeptide selon l'invention modifié par une alanine

<400> 31

His Ala Ala Glu
1

<210> 32
<211> 4
<212> PRT
<213> artificial sequence

<220>

<223> Tétrapeptide selon l'invention modifié par une alanine

<400> 32

```
                                        Ala Ala Asp Asp
                                        1
```

<210> 33
<211> 4
<212> PRT
<213> artificial sequence

<220>
<223> Tétrapeptide selon l'invention modifié par une alanine

<400> 33

```
                                        His Ala Ala Asp
                                        1
```

<210> 34
<211> 4
<212> PRT
<213> artificial sequence

<220>
<223> Tétrapeptide selon l'invention modifié par une alanine

<400> 34

```
                                        His Ala Asp Ala
                                        1
```

<210> 35
<211> 4
<212> PRT
<213> artificial sequence

<220>
<223> Tétrapeptide selon l'invention modifié par une alanine

<400> 35

```
                                        Ala Ala Asp Glu
                                        1
```

<210> 36
<211> 4
<212> PRT
<213> artificial sequence

<220>
<223> Tétrapeptide selon l'invention modifié par une alanine

<400> 36

```
                                        Arg Ala Asp Ala
                                        1
```

<210> 37
<211> 4
<212> PRT
<213> artificial sequence

<220>
<223> Tétrapeptide selon l'invention modifié par une alanine

<400> 37

```
                              Arg Ala Ala Glu
                              1
```

<210> 38
<211> 4
<212> PRT
<213> artificial sequence

<220>
<223> Tétrapeptide selon l'invention modifié par une alanine

<400> 38

```
                              Lys Ala Ala Glu
                              1
```

<210> 39
<211> 6
<212> PRT
<213> artificial sequence

<220>
<223> Tétrapeptide SEQ. ID. n°19 avec 2 acides aminés supplémentaires

<400> 39

```
                    His Ala Glu Glu Ser Asp
                    1                   5
```

<210> 40
<211> 6
<212> PRT
<213> artificial sequence

<220>
<223> Tétrapeptide SEQ. ID. n°19 avec 2 acides aminés supplémentaires

<400> 40

```
                    His Ala Glu Glu Ala Asp
                    1                   5
```

<210> 41
<211> 6
<212> PRT
<213> artificial sequence

<220>

<223> Tétrapeptide SEQ. ID. n°19 avec 2 acides aminés supplémentaires

<400> 41

```
His Ala Glu Glu Ser Glu
1               5
```

<210> 42
<211> 6
<212> PRT
<213> artificial sequence

<220>
<223> Tétrapeptide SEQ. ID. n°19 avec 2 acides aminés supplémentaires

<400> 42

```
His Ala Glu Glu Gly Glu
1               5
```

<210> 43
<211> 6
<212> PRT
<213> artificial sequence

<220>
<223> Tétrapeptide SEQ. ID. n°19 avec 2 acides aminés supplémentaires

<400> 43

```
His Ala Glu Glu Gln Glu
1               5
```

<210> 44
<211> 6
<212> PRT
<213> artificial sequence

<220>
<223> Tétrapeptide SEQ. ID. n°19 avec 2 acides aminés supplémentaires

<400> 44

```
Leu Ala His Ala Glu Glu
1               5
```

<210> 45
<211> 6
<212> PRT
<213> artificial sequence

<220>
<223> Tétrapeptide SEQ. ID. n°19 avec 2 acides aminés supplémentaires

<400> 45

```
Ile Ala His Ala Glu Glu
1               5
```

<210> 46
<211> 6
<212> PRT
<213> artificial sequence

<220>
<223> Tétrapeptide SEQ. ID. n°19 avec 2 acides aminés supplémentaires

<400> 46

```
Phe Ser His Ala Glu Glu
1               5
```

<210> 47
<211> 6
<212> PRT
<213> artificial sequence

<220>
<223> Tétrapeptide SEQ. ID. n°19 avec 2 acides aminés supplémentaires

<400> 47

```
Leu Gly His Ala Glu Glu
1               5
```

<210> 48
<211> 6
<212> PRT
<213> artificial sequence

<220>
<223> Tétrapeptide SEQ. ID. n°19 avec 2 acides aminés supplémentaires

<400> 48

```
Ile Ser His Ala Glu Glu
1               5
```

<210> 49
<211> 10
<212> PRT
<213> artificial sequence

<220>
<223> Tétrapeptide SEQ. ID. n°19 avec 6 acides aminés supplémentaires

<400> 49

```
His Ser Leu Ala His Ala Glu Glu Ser Asp
1               5                   10
```

<210> 50
<211> 10
<212> PRT
<213> artificial sequence

<220>

<223> Tétrapeptide SEQ. ID. n°19 avec 6 acides aminés supplémentaires

<400> 50

```
                              Lys Asn Ile Ala His Ala Glu Glu Ala Asp
                              1               5                   10
```

<210> 51
<211> 10
<212> PRT
<213> artificial sequence

<220>
<223> Tétrapeptide SEQ. ID. n°19 avec 6 acides aminés supplémentaires

<400> 51

```
                              Arg Asn Phe Ser His Ala Glu Glu Ser Glu
                              1               5                   10
```

<210> 52
<211> 10
<212> PRT
<213> artificial sequence

<220>
<223> Tétrapeptide SEQ. ID. n°19 avec 6 acides aminés supplémentaires

<400> 52

```
                              Lys Gln Leu Gly His Ala Glu Glu Gly Glu
                              1               5                   10
```

<210> 53
<211> 10
<212> PRT
<213> artificial sequence

<220>
<223> Tétrapeptide SEQ. ID. n°19 avec 6 acides aminés supplémentaires

<400> 53

```
                              Arg Gln Ile Ser His Ala Glu Glu Gln Glu
                              1               5                   10
```

<210> 54
<211> 10
<212> PRT
<213> artificial sequence

<220>
<223> Tétrapeptide SEQ. ID. n°19 avec 6 acides aminés supplémentaires

<400> 54

```
                              Asp Asp His Ser Leu Ala His Ala Glu Glu
                              1               5                   10
```

<210> 55
<211> 10
<212> PRT
<213> artificial sequence

<220>
<223> Tétrapeptide SEQ. ID. n°19 avec 6 acides aminés supplémentaires

<400> 55

```
Glu Glu Lys Asn Ile Ala His Ala Glu Glu
1               5                   10
```

<210> 56
<211> 10
<212> PRT
<213> artificial sequence

<220>
<223> Tétrapeptide SEQ. ID. n°19 avec 6 acides aminés supplémentaires

<400> 56

```
Glu Glu Arg Asn Phe Ser His Ala Glu Glu
1               5                   10
```

<210> 57
<211> 10
<212> PRT
<213> artificial sequence

<220>
<223> Tétrapeptide SEQ. ID. n°19 avec 6 acides aminés supplémentaires

<400> 57

```
Asp Glu Lys Gln Leu Gly His Ala Glu Glu
1               5                   10
```

<210> 58
<211> 10
<212> PRT
<213> artificial sequence

<220>
<223> Tétrapeptide SEQ. ID. n°19 avec 6 acides aminés supplémentaires

<400> 58

```
Asp Glu Arg Gln Ile Ser His Ala Glu Glu
1               5                   10
```

<210> 59
<211> 3
<212> PRT
<213> artificial sequence

<220>

<223> Comporte une partie du tétrapeptide selon SEQ. ID. n°19

<400> 59

Ala Glu Glu
1

<210> 60
<211> 3
<212> PRT
<213> artificial sequence

<220>
<223> Comporte une partie du tétrapeptide selon SEQ. ID. n°19

<400> 60

His Ala Glu
1

<210> 61
<211> 3
<212> PRT
<213> artificial sequence

<220>
<223> Comporte une partie du tétrapeptide selon SEQ. ID. n°19

<400> 61

Ser His Ala
1

<210> 62
<211> 3
<212> PRT
<213> artificial sequence

<220>
<223> Comporte une partie du tétrapeptide selon SEQ. ID. n°19

<400> 62

Gly His Ala

1

<210> 63
<211> 3
<212> PRT
<213> artificial sequence

<220>
<223> Comporte une partie du tétrapeptide selon SEQ. ID. n°19

<400> 63

```
                                        Glu Glu Gln
                                        1
```

<210> 64
<211> 3
<212> PRT
<213> artificial sequence

<220>
<223> Comporte une partie du tétrapeptide selon SEQ. ID. n°19

<400> 64

```
                                        Ala His Ala
                                        1
```

<210> 65
<211> 3
<212> PRT
<213> artificial sequence

<220>
<223> Comporte une partie du tétrapeptide selon SEQ. ID. n°19

<400> 65

```
                                        Ile Ala His
                                        1
```

<210> 66
<211> 3
<212> PRT
<213> artificial sequence

<220>
<223> Comporte une partie du tétrapeptide selon SEQ. ID. n°19

<400> 66

```
                                        Phe Ser His
                                        1
```

<210> 67
<211> 3
<212> PRT
<213> artificial sequence

<220>
<223> Comporte une partie du tétrapeptide selon SEQ. ID. n°19

<400> 67

```
                                        Glu Ser Asp
                                        1
```

<210> 68
<211> 3
<212> PRT

<213> artificial sequence

<220>
<223> Comporte une partie du tétrapeptide selon SEQ. ID. n°19

<400> 68

```
Ile Ser His
1
```

**Description**

La présente invention concerne un nouveau composé peptidique utile, notamment, pour se lier aux peptides amyloïdes β et/ou empêcher ou réduire la formation de plaques amyloïdes, notamment en inhibant la polymérisation dudit peptide amyloïde sous forme de plaques amyloïdes.

L'invention est définie par les revendications. Tout objet non couvert par la portée des revendications est mentionné/cité uniquement pour information. Toute référence dans la description aux méthodes de traitement se rapporte aux composés, aux compositions pharmaceutiques et aux médicaments de la présente invention destinés à être utilisés dans une méthode de traitement thérapeutique du corps humain ou animal.

La présente invention concerne également une utilisation à titre de médicament ou pour la préparation ou la détermination d'un médicament en vue du traitement d'une maladie neurodégénérative, notamment la maladie d'Alzheimer.

La maladie d'Alzheimer est une maladie neurodégénérative, notamment des personnes âgées, se caractérisant par un long et irréversible déclin des capacités mentales, notamment sur une période de 5 à 15 ans en début de maladie, et, dans les stades ultérieurs de la maladie, une perte des fonctions psychomotrices.

La maladie d'Alzheimer est l'une des plus fréquentes causes de démence parmi les personnes âgées de plus de 55 ans, quelque soit le genre ou le niveau de vie [1].

Pour certains patients, on peut lier la maladie à des disfonctionnements héréditaires mais, pour la majorité des patients, la maladie intervient de façon sporadique, sans aucune raison identifiable [2].

A ce jour, il n'existe pas méthode thérapeutique pour traiter les formes sporadiques ou héréditaires de la maladie. Il est maintenant bien connu que la maladie d'Alzheimer n'est pas une conséquence inévitable du processus de vieillissement mais est induite par un phénomène anormal d'agrégation de peptides amyloïdes-β (ci-après "Aβ") [3, 4].

Aβ est un peptide de 39 à 43 acides aminés (SwissProt ID P05067) qui se forme après clivage séquentiel d'une protéine précurseur de l'amyloïde (ci-après désignée "APP"). Le peptide Aβ est un composé que l'on retrouve communément dans les fluides biologiques, tel que le sang ou le liquide cérébrospinal, dans lesquels il est présent à des concentrations faibles de 0,5 à 5 nM et ce de manière identique chez des individus sains ou des patients atteints de la maladie d'Alzheimer [5, 6].

En revanche, la progression de la maladie d'Alzheimer se caractérise par une accumulation ou dépôt extracellulaire dans le cerveau du patient de plaques dites amyloïdes. Ces plaques résultent de la conformation du monomère de peptide Aβ sous forme de dimères puis d'oligomères solubles de plus grandes tailles, puis leur agrégation sous forme d'agrégats de feuillets β fibrillaires insolubles, qui donnent lieu finalement à des plaques amyloïdes [7].

Toutefois, le mécanisme moléculaire exact du phénomène de formation des plaques amyloïdes et du déclenchement de la maladie d'Alzheimer reste en partie non élucidé à ce jour.

Certaines études imputent un potentiel amyloïdogénique intrinsèquement élevé aux fragments des acides aminés 17-42 de l'extrémité COOH du peptide Aβ [9]. D'autres études n'excluent pas que les fragments des acides aminés 1-16 du peptide Aβ de la région de l'extrémité $NH_2$ du peptide Aβ soient impliqués dans la pathogénèse de la maladie d'Alzheimer [10].

A ce jour, l'ensemble des données disponibles permet de considérer que la région N-terminal 1-16 de peptide Aβ est certainement la région impliquée de façon la plus déterminante et décisive dans le phénomène de formation des plaques amyloïdes pour les raisons suivantes.

Tout d'abord, cette région comporte trois mutations chez le rat, distinguant le peptide Aβ du rat de celui des autres mammifères, alors même que le rat ne connaît pas la maladie d'Alzheimer.

La région N-terminal 1-16 était identifiée comme le domaine de liaison du zinc (Zn) et cuivre (Cu) du peptide Aβ [11-14]. Or, les plaques amyloïdes sont précisément caractérisées par des concentrations élevées anormales de métaux divalents et, en particulier, de zinc [15]. Plusieurs études et expériences, in vitro aussi bien que in vivo, montrent que des concentrations élevées de zinc induisent la précipitation du peptide Aβ, conduisant à la formation de plaques amyloïdes. De même, il a été démontré que des plaques amyloïdes se formaient de façon plus importante dans l'environnement de neurones présentant des concentrations de zinc élevées [16-20].

C'est pourquoi, il est considéré maintenant que le blocage de l'interaction du peptide Aβ avec le zinc doit conduire à une diminution de l'agrégation sous forme de plaques amyloïdes du peptide Aβ et prévenir ainsi les pathologies qui y sont associées. Ainsi, dans US 6 001 852, on propose, à titre de médicament pour traiter la maladie d'Alzheimer, le CLIOQUINOL qui est un agent chélatant le zinc. Toutefois, l'utilisation d'agent chélateur du zinc demande un ciblage spécifique dans la région du domaine de liaison au zinc du peptide Aβ pour éviter l'interférence avec les homéostases normales du zinc dans l'organisme.

D'autres études [21-30] suggèrent que les récepteurs de l'acétylcholine nicotinique jouent un rôle comme cible principale de la neurotoxicité en liaison avec les défaillances du système cholinergique et cognitive induites par la formation de plaques amyloïdes. C'est pourquoi, dans US 7 018 797, on a proposé une méthode de traitement des maladies neurodégénératives par inhibition de la liaison des peptides d'amyloïdes β au récepteur d'acétylcholine nicotinique α-7. Dans US 7 018 797, on propose plus particulièrement, comme composés susceptibles d'inhiber la liaison du peptide à Aβ avec ledit récepteur de l'acétylcholine nicotinique α-7, des composés dérivés du naphtalène, en particulier le 5,8-dihydroxy-trans-2-di(N-propylamino)-3-méthyl-1,2,3,4-tétrahydronaphtalène.

D'autres études ont montré une interaction de la région des acides aminés 13-16 du peptide Aβ (HHQK, SEQ. ID. n°27) avec des cellules microgliales du cerveau par liaison avec l'heparan sulfate, qui est une molécule de liaison desdites cellules rentrant dans la catégorie des glycosaminoglycannes. La liaison du peptide Aβ sur les molécules glycosaminoglycannes de surface des cellules microgliales induit la formation des oligomères de peptides Aβ. La molécule Tramiprosate mime les glycosaminoglycannes et inhibe le processus d'oligomérisation des peptides Aβ [31-36].

Dans US 7 314 724, on propose d'utiliser, comme médicament contre la maladie d'Alzheimer, les formes solubles des laminines et ses fragments (>10 Kda), qui sont capable de se lier avec Aβ et de le garder en état monomère.

Le mécanisme plus précis d'interaction entre les peptides Aβ et les récepteurs d'acétylcholine nicotinique, ainsi que les laminines, n'a pas été élucidé.

Le brevet WO 93/13789 de SmithKline Beecham décrit des composés de formule complexe incluant des oligopeptides protégés de deux cotés dont la formule générale couvre deux hexa ou heptapeptides réunis entre eux par un lien chimiques. L'utilisation des composés décrits dans ce document concerne la stimulation du système d'hémopoïése pour protéger l'organisme contre les infections, plus particulièrement la stimulation du système myélopoïétique et n'explicite pas le mode d'action des dits composés.

US 2004/214165 décrit le système d'export des protéines des mycobactéries. La séquence 646 des 4 acides aminés HAED est juste une séquence d'une partie terminale d'une des protéines de mycobactéries qui participe dans le processus d'export des protéines. Mais, cette séquence HAED n'est pas décrite dans US 2004/214165 en tant que formule d'un oligopeptide isolé individuellement et encore moins, a fortiori, comme un peptide thérapeutiquement actif.

EP 2 130 550 décrit une séquence DEAH (SEQ ID N°39) comme étant un motif d'une protéine ARN hélicase, laquelle présente une activité dans la production des cytokines par cellules mononucléaires du sang. Mais, EP 2 130 550 ne décrit pas un tétrapeptide DEAH en tant que composé isolé et, a fortiori, en tant que composé protégé et, a fortiori encore, en tant que composé actif en lui-même.

L'article de Gomez-Ruiz et al dans J.Dairy Sci. 90 :4966-4973 de 2007 décrit le peptide EDAK en tant que produit que l'on retrouve dans le fromage provenant de la dégradation de protéines du lait causée par la fermentation par des enzymes des lactobacilles ou des enzyme qui ont été artificiellement ajoutés dans le fromage. Cet article ne décrit ni ne suggère que ce tétrapeptide isolé, purifié et, a fortiori, protégé par des groupes protecteurs, c'est-à-dire en dehors de son mélange au fromage, puisse être un composé d'intérêt pris séparément ni, a fortiori, puisse être un composé actif en lui-même.

L'article de Chu et al dans J.Am. Chem. Soc. 1995, 117, 5419-54205 décrit une méthode d'identification de tétrapeptides par spectrométrie. Ces tétrapeptides sont identifiés comme se liant à un récepteur, à savoir la vancomycine, seule l'extrémité N-terminal du tétrapeptide étant protégée. Le tétrapeptide DDAH est juste un des peptides utilisés dans l'expérimentation, choisi pour démontrer les résultats d'une manipulation. L'article ne décrit pas une activité particulière de ce tétrapeptide DDAH.

Le but de la présente invention est de fournir un nouveau composé apte à servir à la détermination ou fabrication d'un médicament pour le traitement ou la prévention des maladies liées à des désordres neurodégénératifs et/ou à la formation de plaques amyloïdes par polymérisation ou agrégation de peptides amyloïdes β.

Plus particulièrement, un but de la présente invention est de fournir un nouveau composé apte à inhiber l'interaction du peptide amyloïde β avec des ions Zn(II). On sait, en effet, que la formation de plaques amyloïdes par agrégation ou polymérisation des peptides amyloïdes β est induite par la formation de complexes du zinc avec le peptide amyloïde β, le zinc agissant en quelque sorte comme cofacteur de cette agrégation ou polymérisation.

Plus particulièrement, le but de la présente invention est de fournir un nouveau composé apte à inhiber la formation de plaques amyloïdes β et permettre le traitement ou la prévention de la maladie d'Alzheimer ou autre maladie dégénérative liée à la formation de plaques amyloïdes.

Pour ce faire, la présente invention fournit un composé peptidique de formule générale suivante (I) ou (II):

$$R^a\text{-}R^1\text{-}R^2\text{-}R^3\text{-}R^4\text{-}R^b \qquad (I)$$

ou

$$R^a\text{-}R^4\text{-}R^3\text{-}R^2\text{-}R^1\text{-}R^b \qquad (II)$$

Dans lesquelles,

- $R^1$ est un reste d'acide aminé H, R ou K;

- R$^2$ est un reste d'acide aminé A;

- R$^3$ est un reste d'acide amine E ou D;

- R$^4$ est un reste d'acide amine E ou D

- R$^a$ représente la fonction amine primaire N-terminale de l'acide aminé R$^1$ ou R$^4$, libre ou substituée par un groupe protecteur, de préférence un groupe acétyle (Ac),

- R$^b$ représente la fonction hydroxyle du groupe carboxyle C-terminale de l'acide aminé R$^1$ ou R$^4$, libre ou substituée par un groupe protecteur, de préférence un groupe NH$_2$, NHR ou NRR avec R représentant une chaine alkyle de C1 à C4, c'est-à-dire correspondant à un groupe de type amide, de préférence un groupe amide (NH$_2$),

On comprend que les composés peptidiques selon la présente invention s'entendent de composés isolés individuellement et purifiés, de préférence avec un degré de pureté supérieur à 98%, de préférence d'au moins 99%, le reste pouvant être constitué d'autres peptides entre autres.

De façon connue et de préférence, les acides aminés R$^1$, R$^2$, R$^3$, R$^4$ seront configurés sous forme de leurs isomères naturel L. Mais, les acides aminés R$^1$, R$^2$, R$^3$ et R$^4$, pourront aussi être des analogues de configuration D.

De préférence encore, un composé peptidique selon la présente invention présentera un degré de pureté en isomères L supérieur à 98%, de préférence d'au moins 99% en isomères L.

Les inventeurs ont déterminé ces tétrapeptides en définissant les différents acides aminés en fonction d'une liaison du tétrapeptide avec le site EVHH (SEQ. ID. n°28) de la région 11-14 de l'amyloïde β, en définissant des acides aminés R$^1$, R$^3$ et R$^4$ ioniquement de signe contraire, c'est-à-dire aptes à former des liaisons ioniques fortes avec les acides aminés E (glutamate) à la position 11 pour R$^1$ et, respectivement, H (histidine) aux positions 13 et 14 pour R$^3$ et, respectivement, R$^4$, c'est-à-dire 3 des sites potentiellement impliqués dans la liaison des ions Zn(II) avec l'amyloïde β, et un acide amine hydrophobe A (alanine) apte à former un effet hydrophobe le plus fort avec V.

Les tétrapeptides ainsi définis forment des interactions non covalentes, parallèles (formule I) et antiparallèles (formule II), avec la cible EVHH de la région 11-14 de l'amyloïde β. Prenant en compte le fait que trois des quatre acides aminés de la région cible EVHH cible présentent des groupements fortement chargés, on a privilégié des acides aminés présentant une complémentarité ioniques pour ces trois acides aminés, tandis que le choix de l'acide aminé R$^2$ a été sélectionné en fonction de considération de complémentarité stérique entre les chaînes latérales de l'alanine et la valine, stabilisant encore davantage, par contact hydrophobe entre V et A, les liaisons ioniques avec E et H.

Les inventeurs ont démontré expérimentalement que des tétrapeptides, tels que définis ci-dessus, se liant à 3 chélateurs des ions Zn (II), à savoir les acides aminés aux positions 11, 13 et 14, représentaient, par comparaison avec d'autres cibles pertinentes de différentes tailles comprenant 1 à 4 chélateurs de Zn(II) choisis parmi les positions 6, 11, 13 et 14, notamment des régions 9-11, 10-12, 11-13, 12-14, 13-15, 14-16, 11-16, 5-14 et 9-14, et d'autres critères de définition, notamment en terme de taille du peptide, représentaient un compromis optimal de possibilité d'utilisation du dit composé in vivo pour l'inhibition de la liaison des ions Zn(II) avec le peptide amyloïde β, (Aβ) et l'inhibition de l'oligomérisation du peptide amyloïde β, par liaison du tétrapeptide avec le domaine de liaison aux ions Zn(II) du peptide amyloïde β (Aβ).

Il apparaît que la force de l'interaction peut être compromise par une liaison au niveau de la position 6. Inversement, une cible ne couvrant que 2 sites aux positions 6 et 11 ou 13 et 14 est insuffisante pour inhiber la liaison des ions Zn(II).

Un autre avantage des tétrapeptides selon l'invention est qu'ils sont fortement électriquement chargés ce qui favorise la capacité de traverser la barrière hémato-encéphalique.

Les composés peptidiques selon l'invention comprennent donc, notamment, les séquences R$^1$-R$^2$-R$^3$-R$^4$ et R$^4$-R$^3$-R$^2$-R$^1$ des formules I et II explicités dans le tableau 1, ci-après, et reprises dans les séquences SEQ. ID. n°1 à 24 du listage de séquences annexé à la description.

Tableau 1 :

| SEQ. ID. n°1 = HADD |
|---|
| SEQ. ID. n°2 = DDAH |
| SEQ. ID. n°3 = KADD |
| SEQ. ID. n°4 = DDAK |
| SEQ. ID. n°5 = RADD |

(suite)

| |
|---|
| SEQ. ID. n°6 = DDAR |
| SEQ. ID. n°7 = HAED |
| SEQ. ID. n°8 = DEAH |
| SEQ. ID. n°9 = KAED |
| SEQ. ID. n°10 = DEAK |
| SEQ. ID. n°11 = RAED |
| SEQ. ID. n°12 = DEAR |
| SEQ. ID. n°13 = HADE |
| SEQ. ID. n°14 = EDAH |
| SEQ. ID. n°15 = KADE |
| SEQ. ID. n°16 = EDAK |
| SEQ. ID. n°17 = RADE |
| SEQ. ID. n°18 = EDAR |
| SEQ. ID. n°19 = HAEE |
| SEQ. ID. n°20 = EEAH |
| SEQ. ID. n°21 = KAEE |
| SEQ. ID. n°22 = EEAK |
| SEQ. ID. n°23 = RAEE |
| SEQ. ID. n°24 = EEAR |

L'affinité de ces tétrapeptides avec un ligand correspondant au fragment de la région 1-16 du peptide amyloïde β a été testée par le biais du calcul de la constante de dissociation Kd aux exemples 1 à 3. Leurs constantes de dissociation sont inférieures à celles d'autres peptides tétrapeptides, tripeptides, hexapeptides ou décapeptides testés ne rentrant pas dans cette définition d'un facteur $10^4$.

De façon connue, lesdits groupes protecteurs sont des groupes compatibles avec une utilisation pharmaceutique in vivo. La fonction des groupes protecteurs est de neutraliser la charge électrique des extrémités amino et carboxy du peptide. Plus particulièrement, l'extrémité N-terminal peut être protégée par un groupe formyle (HCO-) ou acétyle ($CH_3CO$-), et l'extrémité C-terminal peut être protégée par un groupe -$NH_2$, -NHR, -NRR, avec R un alkyle en C1 à C4, ou encore -O-R, avec R un alkyle en C1 à C4 ou un alkylamine, tel que -$OCH_3$, -$OCH_2CH_3$ ou -$OCH_2CH_2NH_2$.

On rappelle que :

- les acides aminés hydrophobes à groupement apolaire sont la l'alanine (A), la valine (V), la leucine (L), l'isoleucine (I), la méthionine (M), la phénylalanine (F), la proline (P), et le tryptophane (W),

- les acides aminés hydrophiles à groupement polaire sont la glycine (G), la sérine (S), la thréonine (T), la cystéine (C), la tyrosine (Y), l'asparagine (N) et la glutamine (Q),

- les acides aminés à groupement chargé négativement à pH neutre (acide) sont l'acide aspartique (D) et l'acide glutamique (E), et

- les acides aminés à groupe chargés positivement à pH neutre (basique) sont la lysine (K), l'arginine (R) et l'histidine (H).

Les acides α aminés naturels sont tous chiraux, donc optiquement actifs, à l'exception de la glycine, et sont tous de configuration L selon la configuration de FISHER, ou S selon la notation de CAHN-INGOLD-PROLOG.

Trois grands types d'interaction interviennent dans les liaisons entre peptides ou les structures secondaires des peptides de type hélice α ou feuillet plissé β résultant de la formation de ponts hydrogènes entre les groupements CO et NH du squelette peptidique, à savoir :

- l'effet hydrophobe : les acides aminés, dont les radicaux sont hydrophobes, ont plus d'affinité entre eux qu'avec des molécules d'eau entourant la protéine. Inversement, les acides aminés hydrophiles ont tendance à se disposer de façon à être en contact avec l'eau,

- les liaisons ioniques : les radicaux, qui sont chargés positivement, forment des liaisons ioniques avec ceux qui sont chargés négativement, et

- les liaisons hydrogènes qui ont des interactions moins fortes que celles résultant des liaisons ioniques et par effet hydrophobe.

De préférence, le composé peptidique selon l'invention sera choisi parmi les composés de formule (I) ou (II) dans lesquelles $R^1$-$R^2$-$R^3$-$R^4$ ou, respectivement, $R^4$-$R^3$-$R^2$-$R^1$ représentent HAEE, EDAR, EEAH, EEAK, EEAR, HADE, KADD, KAEE, RADD, RADE, RAEE, HADD, DDAK, KAED, DEAR ou KADE. Ces peptides correspondent dans un ordre différent aux séquences SEQ. ID. n°1, 3, 4, 5, 9, 12, 13, 15, 17, 18, 19, 20, 21, 22, 23 et 24.

Les tétrapeptides ci-dessus présentaient une constante de dissociation de leur interaction avec le fragment de la région 1-16 de l'amyloïde β, Kd telle que calculée aux exemples 1 à 3 ci-après, plus faible que pour les autres peptides testés.

De préférence encore, le composé peptidique selon l'invention parmi les composés de formule (I) ou (II), dans lesquels $R^1$-$R^2$-$R^3$-$R^4$ ou $R^4$-$R^3$-$R^2$-$R^1$ représentent l'un des tétrapeptides HADD, DDAK, RADD, KAED, DEAR, KADE, HAEE et RAEE. Ces peptides correspondent aux séquences SEQ. ID. n°1, 4, 5, 9, 12, 15, 19 et, respectivement, 23. Ils présentent une constante de dissociation Kd plus faible encore que celles des autres peptides testés, telle que calculée aux exemples 1 à 3.

De préférence encore, le composé peptidique selon l'invention est choisi parmi les composés de formule (I) ou (II), dans lesquels $R^1$-$R^2$-$R^3$-$R^4$ ou $R^4$-$R^3$-$R^2$-$R^1$ représentent l'un des deux tétrapeptides HAEE et RADD.

- $R^1$ est un résidu d'acide aminé H,

- $R^2$ est un résidu d'acide aminé A;

- $R^3$ est un résidu d'acide amine E,

- $R^4$ est un reste d'acide amine E.

Les composés peptidiques les plus préférés répondent aux formules Ac-HAEE-$NH_2$ et Ac-RADD-$NH_2$.

Les constantes de dissociation d'interaction Kd de ces deux peptides avec le ligand correspondant à un fragment de la région 1-16 de l'amyloïde β, à l'exemple 1, étaient les plus faibles de celles de tous les peptides testés.

Ces deux composés peptiques apparaîssent aussi comme étant les plus performants en termes d'inhibition de la liaison des ions Zn(II) au peptide amyloïde β et inhibition de la polymérisation ou agrégation du peptide amyloïde β sous forme de plaque, en présence de concentrations physiologiques d'ions Zn(II), notamment de 100 à 400 $\mu$M par liaison au dit peptide amyloïde β. Mais, surtout, ces deux composés peptidiques présentaient les propriétés d'inhibition de la formation de plaques amyloïdes dans un modèle in vivo sur souris, les plus performantes, comme rapporté aux exemples ci-après.

La présente invention a également pour objet l'utilisation d'un composé peptidique selon l'invention, pour réduire ou empêcher la liaison des ions Zn(II) au peptide amyloïde β par liaison dudit composé peptidique au dit peptide amyloïde β.

Plus particulièrement encore, la présente invention a pour objet l'utilisation d'un composé peptidique selon l'invention, pour réduire ou empêcher la polymérisation et/ou l'agrégation du peptide amyloïde β, en présence d'ions Zn(II), plus particulièrement à des concentrations physiologiques d'ions Zn(II) notamment de 100 à 400 $\mu$M, par liaison dudit composé peptidique au dit peptide amyloïde β.

La présente invention fournit donc l'utilisation d'un composé peptidique selon l'invention, pour le traitement d'une maladie liée à la formation de plaques amyloïdes.

La présente invention a également pour objet l'utilisation d'un composé peptidique selon l'invention à titre de médicament, notamment pour le traitement d'une maladie neurodégénérative et, de préférence, pour le traitement de la maladie d'Alzheimer.

La présente invention a également pour objet une composition utile dans une utilisation selon l'invention, dont la substance active comprend au moins un reste $R^1$-$R^2$-$R^3$-$R^4$ ou $R^4$-$R^3$-$R^2$-$R^1$ d'un composé peptidique selon l'invention.

La substance active peut être un multimère constitué de plusieurs monomères de séquence $R^1$-$R^2$-$R^3$-$R^4$ ou $R^4$-$R^3$-$R^2$-$R^1$ lié par un lien apte à se dégrader dans l'organisme et ainsi libérer le tétrapeptide de façon prolongée dans le temps le monomère actif.

De façon connue, un composé peptidique selon l'invention pourra être formulé sous forme de dérivés ester ou de sels physiologiquement acceptables.

Plus particulièrement, la présente invention a pour objet une composition pharmaceutique comprenant un composé selon l'invention ou une substance active comprenant au moins un reste $R^1$-$R^2$-$R^3$-$R^4$ ou $R^4$-$R^3$-$R^2$-$R^1$ d'un composé peptidique selon l'invention, avec un véhicule pharmaceutiquement acceptable, de préférence par voie parentérale, transcutanée ou transmuqueuse.

Plus particulièrement, la composition pharmaceutique selon l'invention sera formulée pour être injectable, notamment par voie intravasculaire, intramusculaire, sous-cutanée, intrarachidienne ou cérébrospinale.

D'autres modes d'administration par voie orale, buccale, nasale, rectale ou topique sont envisageables sous réserve de formuler le composé peptidique selon l'invention sous forme apte de résister à la dégradation avant d'atteindre les cellules cibles du cerveau.

De préférence encore, ladite composition se présentera sous forme lyophilisée.

Plus particulièrement, dans une composition selon la présente invention, la concentration en dits composés peptidiques est de 20 à 2 000 μM. Les exemples suivants décrivent et démontrent l'efficacité des composés peptidiques selon l'invention, donnés uniquement à titre d'illustration et ne devant pas être interprétés comme une limitation de la présente invention.

D'autres caractéristiques et avantages de la présente invention apparaitront à la lumière des exemples ci-après.

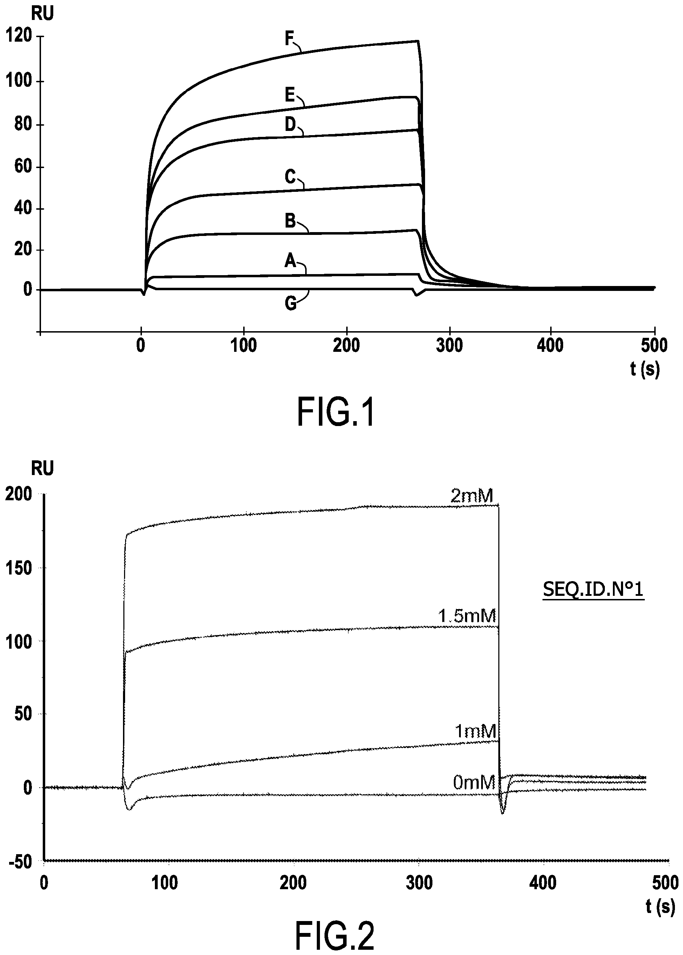

la figure 1 représente des courbes de cinétiques de liaison du peptide Ac-HAEE-NH$_2$ avec un ligand correspondant à la région 1-16 de l'amyloïde β à différentes concentrations, à savoir courbe A=2 μM, courbe B=5 μM, courbe C=10 μM, courbe D=15 μM, courbe E=20 μM, courbe F=25 μM et courbe G=0 μM, avec le matériel et selon le protocole de l'exemple 1.

Les figures 2 à, respectivement, 9 représentent des courbes de cinétiques de liaison des peptides de séquences SEQ. ID. n°1, 4, 5, 9, 12, 15, 19 et, respectivement, 23, avec le matériel et selon le protocole de l'exemple 3, lesdits tétrapeptides étant davantage purifiés qu'à l'exemple 1.

La variation de l'unité de réponse du spectromètre en ordonnées (RU = Unité de réponse) dans le temps permet de calculer la constante de dissociation Kd de la liaison du tétrapeptide avec le fragment de la région 1-16 du peptide amyloïde β, tel que rapporté aux exemples 1 et 3.

Exemple 1 : liaison du tétrapeptide Ac-HAEE-NH$_2$ (SEQ. ID. n°19) à un fragment peptidique immobilisé correspondant à la région 1-16 du peptide amyloïde β.

On a réalisé une analyse par spectroscopie de résonnance des plasmons de surface (SPR) de la liaison du tétrapeptide Ac-HAEE-NH$_2$ à un fragment peptidique immobilisé correspondant à la région 1-16 du peptide amyloïde β.

Cette analyse permet de calculer la constante de dissociation Kd de l'interaction du peptide avec le ligand immobilisé, étant entendu que la valeur de Kd est d'autant plus faible que l'interaction avec le ligand et donc la stabilité du complexe d'interaction formé sont élevées. On considère, dans ces méthodes, que l'on se trouve en présence d'une interaction spécifique significative si Kd est inférieur ou égal à $10^{-4}$ M. En revanche, si Kd est supérieur ou égal à $10^{-3}$ M, on considère qu'il n'y a pas de liaison ou d'interaction significative.

La résonnance des plasmons de surface est un phénomène physique, principalement connu pour son utilisation comme méthode de mesure de la liaison d'un ligand sur un récepteur adsorbé à la surface d'une couche métallique. Un système de détection SPR mesure la variation de l'indice de réfraction au voisinage de l'interface quand le ligand se fixe au récepteur.

Les principes de spectroscopie par résonnance de plasmons de surface sont décrits dans Nagata, K., and Handa, H. (eds), Speinger-Verlag, Tokyo, 2000.

Plus précisément, dans un système de spectroscopie SPR, par exemple mettant en œuvre un biocapteur de la société BIACORE, après immobilisation du ligand à la surface d'un capteur de type puce, en l'espèce ici le fragment d'amyloïde β, le réactif analyte est injecté sur ladite surface à débit constant à travers des systèmes de canaux micro fluidiques. Une lumière polarisée émise par une source de lumière est réfléchie sur la surface couverte d'or de la puce, puis détectée par un lecteur à diode. Selon la liaison du ligand et de la molécule analyte, l'intensité de la lumière réfléchie est différente. La variation de la réponse du spectromètre (RU= Unité de réponse) permet de calculer la constante de dissociation de l'interaction.

Les puces du biocapteur provenaient de chez GE Healthcare (USA). Les expériences de spectroscopie SPR ont été réalisées avec l'appareil BIACORE 3000. Les réactifs pour l'immobilisation du fragment peptidique d'amyloïde β (EDC, NHS, PDEA et cystéine) ont été achetés chez GE Healthcare (USA). Le tampon utilisé pour immobiliser le ligand peptide par formation de liaisons thiol était du sodium acétate 10 mM à pH 4,5. Le tampon de régénération était un tampon HBS contenant du milieu HEPES 10 mM et de l'EDTA 3mM, 0,005% de surfactant P20 et du NaCl 150 mM à pH 7,4. Le tampon d'immobilisation était un tampon HBS à pH 7,4 et un tampon HEPES à pH 6,8, 50 mM, avec 100 $\mu$M d'ions Zn(II) (II) pour le tétrapeptide réactif Ac-HAEE-NH$_2$. Tous les tampons ont été filtrés avant utilisation.

Le fragment peptidique du ligand était : Ac-DAEFRHDSGYEVHHQKGGGGC-NH$_2$, correspondant à la séquence SEQ. ID. n°26. Ce peptide de 21 acides aminés contient les 16 acides aminés de l'extrémité N-terminal du domaine de liaison de l'amyloïde β, à savoir DAEFRHDSGYEVHHQK (SEQ. ID. n°25), suivis de 4 acides aminés Glycine et d'une extrémité C-Terminal cystéine. La cystéine est nécessaire pour immobiliser le ligand sur la surface de la puce par une liaison thiol selon le protocole du fabricant. 4 acides aminés Glycine constituent donc un tétrapeptide de liaison ("linker") entre la puce et le ligand. En outre, le ligand ainsi immobilisé présente assez de flexibilité par rapport à la surface, de façon à mimer valablement les conditions d'interaction entre le réactif tétrapeptide et le ligand. Ce ligand a été fixé sur une puce CM5 du biocapteur, en suivant la procédure standard par liaison thiol dans les conditions décrite dans le manuel du capteur fourni par le fabricant GE Healthcare (USA).

Le débit mis en œuvre dans toutes les étapes était de 5 $\mu$l/min. La matrice de dextran carboxyméthyl a été activée par injection d'un mélange 1:1 de 1-éthyl-3(3-diméthylaminopropyl)-carbodiimide (EDC) et de N-hydroxysuccinimide (NHS), à savoir 30 $\mu$l de EDC à 400 mM et NHS à 100 mM, suivie d'une injection de PDEA (2-(2-pyridinyldithio)éthaneamine) à 80 mM en solution dans du borate de sodium à pH 8,5 à 0,1 M. Le ligand peptide en solution dans un tampon acétate de sodium à raison de 0,05 mg/ml a ensuite été injecté. Les groupes thiol n'ayant pas réagi à la surface des puces CM5, ont été éliminés avec une solution de cystéine à 50 mM dans un tampon acétate de sodium 0,1 M à pH 4, pour fournir une surface qui donnait une variation d'unité de réponse (RU) d'environ 700 unités arbitraires avec un échantillon de liquide contrôle. Le liquide contrôle était mis à réagir avec la surface de la puce selon le même protocole.

Le réactif Ac-HAEE-NH$_2$ a été préparé par dilution selon 7 concentrations, à savoir 0, 2, 5, 10, 15, 20 et 25 $\mu$M, et injecté en mode multicanaux (20 $\mu$l QUICKINJECT, 5 $\mu$l/min). La surface de la puce a été exposée pendant 300 secondes à la solution tampon de réactif pour suivre sa fixation. La surface de la puce a ensuite été régénérée par injection d'un tampon de régénération (20 $\mu$l). Les données de la piste de contrôle ont été soustraites des données brutes de la piste correspondant aux peptides réactifs immobilisés. Ensuite, la constante de dissociation Kd a été calculée de manière connue à partir des courbes établies pour la réponse à l'équilibre (Req) en fonction de la concentration (M) dans le programme d'évaluation BIA 4.1. 7 échantillons de réactif aux différentes concentrations ont donc été injectés dans des canaux avec le ligand immobilisé sur les surfaces de la puce.

Les courbes cinétiques de liaison Ac-HAEE-NH$_2$ aux peptides immobilisés, après soustraction des réponses du témoin de contrôle, sont rapportées sur la figure 1.

Les courbes de la figure 1 ont permis de calculer la constante de dissociation Kd des interactions, qui a été évaluée à $2,2 \times 10^{-8}$ M.

En présence de concentrations physiologiques d'ions Zn(II) (100-400 $\mu$M), la constante de dissociation était pratiquement inchangée. Toutefois, des concentrations élevées d'ions Zn(II) (10 mM) supprimaient les interactions du ligand avec le réactif. Si l'on prend en compte que les liaisons d'ions Zn(II) aux peptides Aβ se caractérisent par des valeurs micro molaires ($\mu$M) de Kd, on peut estimer que le réactif Ac-HAEE-NH$_2$ rentre en compétition avec les ions Zn(II) pour la liaison sur le peptide Aβ. Cependant, l'affinité du réactif avec le peptide Aβ est beaucoup plus élevée que celle des ions Zn(II), de sorte que le peptide Ac-HAEE-NH$_2$ empêche la liaison des ions Zn(II) aux concentrations physiologiques avec le peptide Aβ.

Plus précisément, l'affinité du réactif Ac-HAEE-NH$_2$ avec l'amyloïde Aβ est supérieure à celle des ions Zn(II).

Les expériences ont démontré qu'en présence de concentrations physiologiques d'ions Zn(II) à des niveaux micro molaires (inférieurs à 500 $\mu$M), la liaison de HAEE au domaine 1-16 de l'amyloïde β n'est pratiquement pas altérée en présence ou en l'absence d'ions Zn(II). En revanche, des concentrations plus élevées d'ions Zn(II), de l'ordre du milli molaire (mM), suppriment les interactions entre HAEE et le domaine 1-16 de Aβ. Ces données indiquent que HAEE possède une affinité supérieure pour le domaine cible que les ions Zn(II), et est capable de bloquer l'interaction entre les ions Zn(II) et Aβ lorsque les ions Zn(II) sont à des concentrations physiologiques.

Exemple 2 : Exemple comparatif

Les expériences de liaison à un ligand peptide cible correspondant à la région 1-16 de l'amyloïde β ont été réalisées conformément au protocole décrit à l'exemple 1 avec d'autres peptides décrits ci-après.

1- Tous les 23 autres tétrapeptides listés dans le tableau 1 ci-dessus, correspondant aux séquences SEQ. ID. n°1 à 18 et 20 à 24 avec les mêmes groupes protecteurs R$^a$ = Ac et R$^b$ = NH$_2$, ont été testés et leurs constantes de dissociation Kd étaient de $10^{-5}$ à $10^{-6}$ M. Ces constantes de dissociation étaient pratiquement inchangées en présence de concentrations physiologiques de zinc de 100 à 400 μM. Les tétrapeptides selon l'invention, présentant la constante de dissociation Kd la plus faible (excepté HAEE), étaient : EDAR, EEAH, EEAK, EEAR, HADE, KADD, KAEE, RADD, RADE, RAEE, HADD, DDAK, KAED, DEAR et KADE.

Ces valeurs correspondent à des liaisons fortes avec le ligand cible de la région 1-16 du peptide amyloïde β.

2- D'autres exemples comparatifs avec 10 différents tétrapeptides, analogues à des tétrapeptides du tableau 1 mais dans lesquels un aminoacide a été remplacé par l'alanine (A), à savoir lesdits tétrapeptides suivants : AAEE, HAEA, HAAE, AADD, HAAD, HADA, AADE, RADA, RAAE, KAAE, correspondant aux séquences SEQ. ID. n°29 à 38, respectivement, présentaient une constante de dissociation nettement supérieure, à savoir Kd de $10^{-3}$ à $10^{-4}$ M, correspondant à une interaction non spécifique ou non significative avec le ligand cible.

SEQ. ID. n°29 est tirée de SEQ. ID. n°23, dans lequel on a remplacé le premier acide aminé R par A.

SEQ. ID. n°30 est tirée de SEQ. ID. n°19, dans lequel on a remplacé le dernier acide aminé E par A.

SEQ. ID. n°31 est tirée de SEQ. ID. n°19, dans lequel on a remplacé le troisième acide aminé E par A.

SEQ. ID. n°32 est tirée de SEQ. ID. n°5, dans lequel on a remplacé le premier acide aminé R par A.

SEQ. ID. n°33 est tirée de SEQ. ID. n°7, dans lequel on a remplacé le troisième acide aminé E par A.

SEQ. ID. n°34 est tirée de SEQ. ID. n°1, dans lequel on a remplacé le dernier acide aminé D par A.

SEQ. ID. n°35 est tirée de SEQ. ID. n°13, dans lequel on a remplacé le premier acide aminé H par A.

SEQ. ID. n°36 est tirée de SEQ. ID. n°5, dans lequel on a remplacé le dernier acide aminé D par A.

SEQ. ID. n°37 est tirée de SEQ. ID. n°23, dans lequel on a remplacé le troisième acide aminé E par A.

SEQ. ID. n°38 est tirée de SEQ. ID. n°21, dans lequel on a remplacé le troisième acide aminé E par A.

3- On a testé 10 différents tripeptides correspondant au tétrapeptide SEQ. ID. n°19 (HAEE), dans lequel on a retiré 1, 2 ou 3 acides aminés selon les séquences SEQ. ID. n°59 à 68 suivantes : AEE, HAE, SHA, GHA, EEQ, AHA, IAH, FSH, ESD, ISH. On notera que le tripeptide AEE correspond à un tripeptide complémentaire de la région 12-14 selon les critères de complémentarité explicités précédemment. Ces tripeptides étaient complémentaires des régions du peptide Aβ 9-11, 10-12, 11-13, 12-14, 13-15 et 14-16.

Ces 10 différents tripeptides présentaient également une constante de dissociation Kd de $10^{-3}$ à $10^{-4}$ M, reflétant une absence d'interaction significative ou spécifique avec la cible.

4- En outre, 10 différents hexapeptides, incluant le tétrapeptide SEQ. ID. n°19 (HAEE) préféré selon l'invention, additionné de 2 acides aminés à son extrémité N terminal ou à son extrémité C terminal, ont été testés, à savoir les hexapeptides suivants : HAEESD, HAEEAD, HAEESE, HAEEGE, HAEEQE, LAHAEE, IAHAEE, FSHAEE, LGHAEE, ISHAEE. Ces hexapeptides étaient complémentaires des régions du peptide Aβ 11-16 et 9-14.

Ces 10 hexapeptides, repris dans le listage de séquences dans les séquences SEQ. ID. n°39 à 48, présentaient également une constante de dissociation relativement élevée Kd de $10^{-3}$ à $10^{-4}$ M, reflétant une absence d'interaction significative ou spécifique avec le ligand de la région 1-16 de l'amyloïde β.

Ces 10 hexapeptides correspondaient tous à des séquences dont les 2 acides aminés supplémentaires étaient susceptibles d'interagir avec les acides aminés 15-16 (QK) de l'amyloïde β pour les extensions N terminal et avec les acides aminés GY des régions 9-10 (GY) de l'amyloïde β, conformément aux interactions ioniques ou hydrophobes explicitées précédemment.

5- Enfin, on a testé 10 décapeptides repris dans le listage de séquences, dans les séquences SEQ. ID. n°49 à 58 suivantes : HSLAHAEESD, KNIAHAEEAD, RNFSHAEESE, KQLGHAEEGE, RQISHAEEQE, DDHSLAHAEE, EEKNIAHAEE, EERNFSHAEE, DEKQLGHAEE, DERQISHAEE, qui présentaient une constante de dissociation Kd de $10^{-3}$ à $10^{-4}$ M, reflétant une absence d'interaction significative ou spécifique avec la cible.

Ces décapeptides reprenaient un tétrapeptide préféré selon l'invention, SEQ. ID. n°19 (HAEE), avec 2 acides aminés supplémentaires à l'extrémité N terminal et 4 acides aminés supplémentaires à l'extrémité C terminal pour SEQ. ID. n°49 à 53, et 6 acides aminés supplémentaires pour les décapeptides SEQ. ID. n°54 à 58. Les décapeptides SEQ. ID. n° 49 à 58 comprenaient tous des acides aminés supplémentaires définis comme étant susceptibles d'interagir avec les acides aminés de la région 7-10 et 15-16 de l'amyloïde β pour SEQ. ID. n°49 à 53, et de la région 5 à 10 de l'amyloïde β pour SEQ. ID. n°54 à 58, lesquels ciblaient la région 5-14 du peptide Aβ.

Tous les peptides testés ci-dessus en 1- à 5- comportaient un groupe protecteur de la fonction amine N terminal, $R^a$=Ac et un groupe protecteur de la fonction hydroxyle du groupement carboxyl C terminal, $R^b$=NH$_2$.

Exemple 3 : liaison des tétrapeptides selon l'invention à un fragment peptidique immobilisé correspondant à la région 1-16 du peptide amyloïde β

On a reproduit les essais de liaison décrits à l'exemple 1, avec les mêmes matériels, réactifs et protocoles, excepté les différences significatives suivantes :

- l'appareil BIACORE mis en œuvre était l'appareil BIACORE T100. Cet appareil BIACORE T100 produit des résultats plus précis que le BIACORE 3000 du fait qu'il comporte un système de dé-gazéification lors de l'injection de l'analyte sur le capteur et que la méthode de détection des complexes intermoléculaires ligands/analytes immobilisés sur la puce CM5 est plus précise.

- les tétrapeptides selon l'invention utilisés étaient purifiés à 99% au moins en isomères L d'après les données HPLC, les isomères D étant empêchés de se former et/ou éliminés lors de la synthèse. Dans l'exemple 1, les tétrapeptides comportaient seulement 98% d'isomères L.

- les concentrations des tétrapeptides selon l'invention mises en œ uvre étaient de 50, 100, 200, 500, 1000, 1500 et 2000 $\mu$M.

- ces expériences ont été réalisées en l'absence de zinc.

- la surface des puces CM5 fournissait une surface qui donnait une variation d'unités de réponse (RU) d'environ 1023 unités arbitraires, avec un échantillon de liquide contrôle.

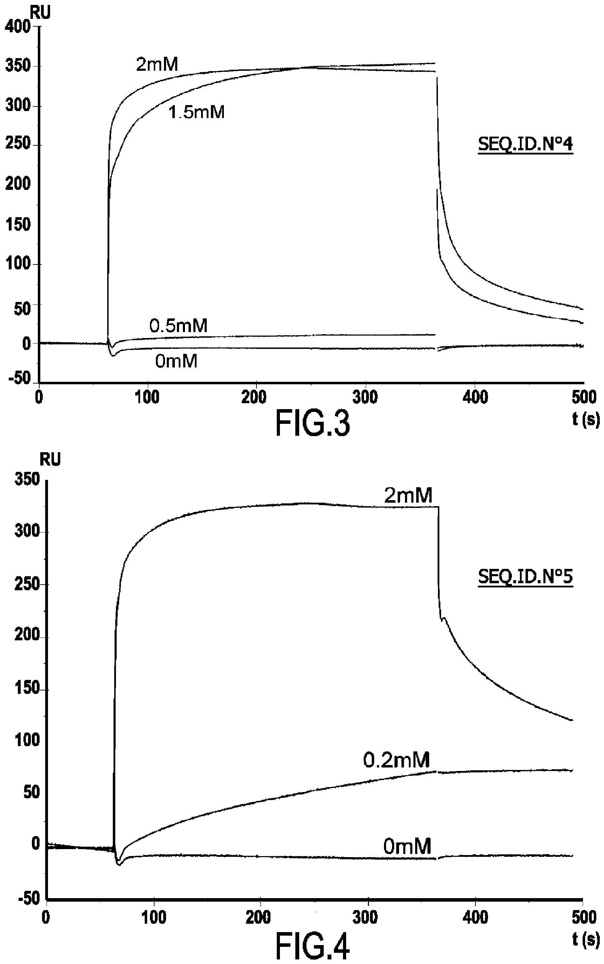

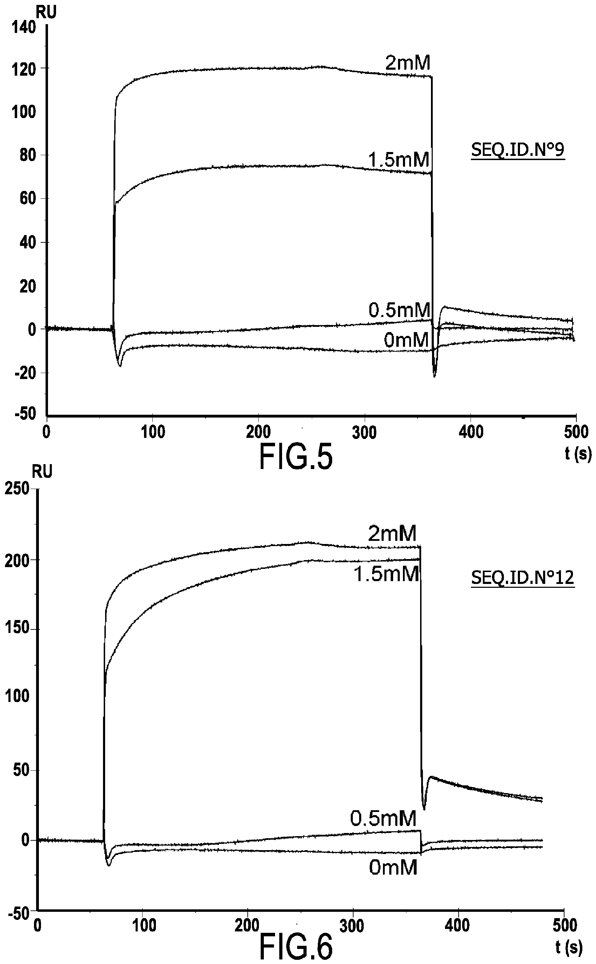

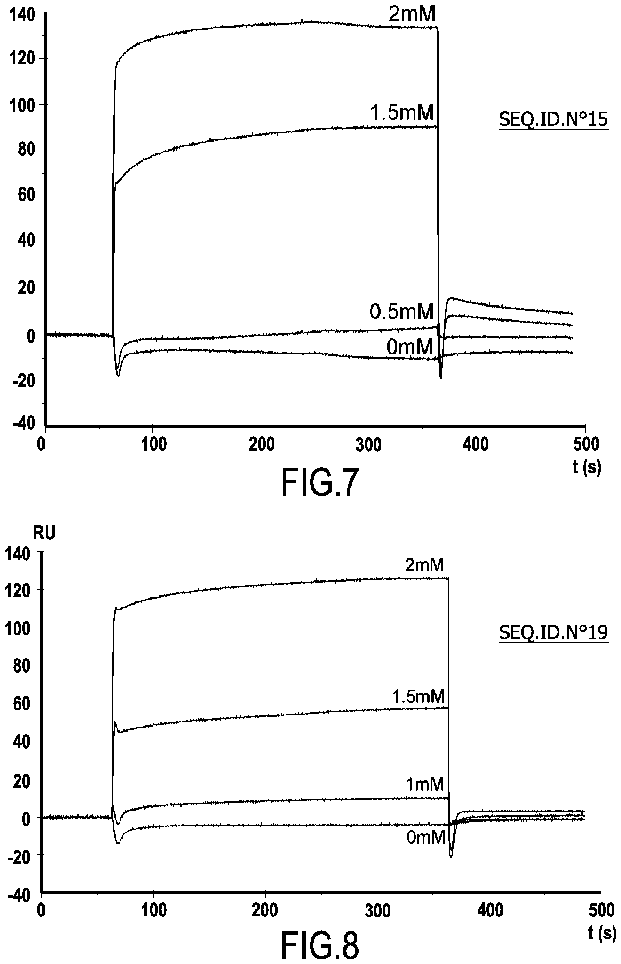

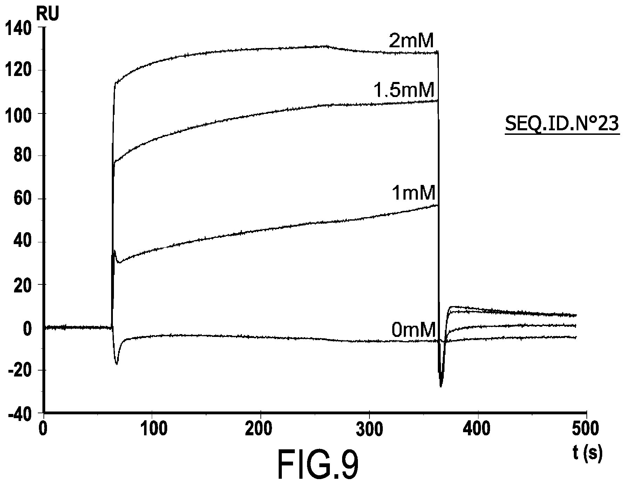

Les courbes des cinétiques des figures 2 à 9 ont permis de calculer la constante de dissociation Kd selon la méthode décrite dans les publications antérieures (voir notamment la référence [39]), à savoir que les courbes permettent de calculer les constantes kon et koff par la formule :

$$dR/dt = k_{on} \, C \, (Rmax - R) - k_{off} \, R,$$

dans laquelle :

- R représente la réponse en ordonnées

- C représente la concentration de l'analyte

- Rmax la valeur maximale théorique de R,

la constante de dissociation Kd étant donnée par la formule Kd = koff/kon.

Dans le tableau 2, sont rapportées les valeurs de constantes de dissociation Kd, calculées pour les tétrapeptides des séquences SEQ. ID. n°1, 4, 5, 9, 12, 15, 19 et 23, qui présentaient les valeurs les plus faibles.

Tableau 2 :

| Analyte | Kd $\times$ 10⁴ M | $k_{on}$ $\times$ M⁻¹s⁻¹ | $k_{off}$ $\times$ 10⁻³ s⁻¹ |
|---|---|---|---|
| SEQ. ID. n°1 | 32.5±0.9 | 3.20±0.08 | 10.40±0.02 |
| SEQ. ID. N°4 | 13.7±0.5 | 4.57±0.09 | 6.2±0.1 |
| SEQ. ID. N°**5** | **0.13±0.02** | **18.5±0.1e1** | **0.24±0.04** |
| SEQ. ID. n°9 | 33±7 | 5.7±0.9 | 19±1 |
| SEQ. ID. n°12 | 19.8±0.7 | 3.58±0.08 | 7.09±0.09 |
| SEQ. ID. n°15 | 45±3 | 2.2±0.1 | 9.6±0.2 |
| SEQ. ID. N°**19** | **0.9±0.3** | **0.37±0.05** | **0.035±0.008** |
| SEQ. ID. n°23 | 77±6 | 1.5±0.1 | 11.20±0.08 |

En l'espèce, les deux analytes comprenant les séquences SEQ. ID. n°5 et 19 présentaient des constantes de dissociation Kd les plus faibles, comprises entre environ 10⁻⁴ et 10⁻⁵ M.

Les six autres tétrapeptides des séquences SEQ. ID. n°4, 9, 12, 15 et 23 donnaient des valeurs de Kd comprises entre 10⁻³ et 10⁻² M.

Les autres tétrapeptides montraient, selon ce modèle, des constantes de dissociation Kd supérieures à 10⁻² M.

Exemple 4 : Inhibition de la formation de plaques amyloïdes β par polymérisation ou agrégation

Le phénomène d'agrégation des peptides Aβ sous forme de plaques amyloïdes a été démontré in vitro en utilisant le kit "SYNTHALOID SCREENING PLATE®" de la société QUALITY CONTROLLED BIOCHEMICALS Inc. USA, impliquant la mise en œuvre de plaques recouvertes de centres de cristallisation du peptide Aβ et de peptides Aβ marqués avec un marquage fluorescent aux fins de détection des agrégats (37, 38).

On a utilisé le peptide β complet 1-40 marqué par un marquage fluorescent dans un tampon contenant du milieu hepes 50 mM, pH 7,4, 0,1% BSA et 10% FCS, ce tampon contenant intrinsèquement des concentrations physiologiques d'ions Zn(II), et des inhibiteurs de protéases. Lesdites plaques étaient sous forme de plaques comportant 96 puits, dans lesquels on a versé 100 μl de peptides Aβ (1-40) marqués à la concentration de 100 nM. On a laissé incuber pendant 3 heures à température ambiante dans les puits.

Dans certains puits, on a introduit, en outre, les tétrapeptides AC-HAEE-NH$_2$ et Ac-RADD-NH$_2$ selon l'invention, à différentes concentrations selon les puits (0, 5, 10, 20, 40, 80, 100 et 150 μM, pour vérifier l'effet des tétrapeptides selon l'invention sur le phénomène d'agrégation des peptides Aβ.

Pour ce faire, à la fin de l'incubation, les protéines non liées dans les puits ont été éliminées par 3 lavages, en utilisant la solution tampon ci-dessus. La quantité de protéines liées sous forme d'agrégats, fixés au puits, était mesurée par mesure de l'intensité de fluorescence.

Les tétrapeptides HAEE et RADD selon l'invention inhibent effectivement l'agrégation du peptide Aβ avec des concentrations inhibitrices IC$_{50}$ de 20 μM. On rappelle que la concentration IC$_{50}$ représente la concentration permettant d'empêcher l'agrégation de 50% des peptides Aβ introduits dans le puits.

Selon le test décrit dans le manuel d'utilisation du kit de plaques "SYNTHALOID SCREENING PLATE®", il est considéré qu'un composé inhibe la liaison sur support solide d'une protéine si sa concentration IC$_{50}$ est inférieure à 100 μM.

Des études dans le temps ont montré qu'une incubation prolongée d'au moins 1 heure des agrégats amyloïdes avec les tétrapeptides HAEE et RADD aux concentrations de 20, 40, 80, 100 et 150 μM provoquait, en outre, une désagrégation irréversible des agrégats amyloïdes.

REFERENCES BIBLIOGRAPHIQUES

[1] Cummings, J.L. (2004). Alzheimer's disease. N Engl J Med 351, 56-67.

[2] Selkoe, D.J. (2001). Alzheimer's disease: genes, proteins, and therapy. Physiol Rev 81, 741-66.

[3] Glenner, G.G. and Wong, C.W. (1984). Alzheimer's disease and Down's syndrome: sharing of a unique cerebrovascular amyloid fibril protein. Biochem Biophys Res Commun 122, 1131-5.

[4] Masters, C.L., Simms, G., Weinman, N.A., Multhaup, G., McDonald, B.L. and Beyreuther, K. (1985). Amyloid plaque core protein in Alzheimer disease and Down syndrome. Proc Natl Acad Sci U S A 82, 4245-9.

[5] Mayeux, R. et al. (1999). Plasma amyloid beta-peptide 1-42 and incipient Alzheimer's disease. Ann Neurol 46, 412-6.

[6] Seubert, P. et al. (1992). Isolation and quantification of soluble Alzheimer's beta-peptide from biological fluids. Nature 359, 325-7.

[7] Hardy, J. and Selkoe, D.J. (2002). The amyloid hypothesis of Alzheimer's disease: progress and problems on the road to therapeutics. Science 297, 353-6.

[8] Luhrs, T., Ritter, C., Adrian, M., Riek-Loher, D., Bohrmann, B., Dobeli, H., Schubert, D. and Riek, R. (2005). 3D structure of Alzheimer's amyloid-beta(1-42) fibrils. Proc Natl Acad Sci U S A 102, 17342-7.

[9] Gowing, E., Roher, A.E., Woods, A.S., Cotter, R.J., Chaney, M., Little, S.P. and Ball, M.J. (1994). Chemical characterization of A beta 17-42 peptide, a component of diffuse amyloid deposits of Alzheimer disease. J Biol Chem 269, 10987-90.

[10] Mattson, M.P. (1995). Untangling the pathophysiochemistry of beta-amyloid. Nat Struct Biol 2, 926-8.

[11] Guilloreau, L., Damian, L., Coppel, Y., Mazarguil, H., Winterhalter, M. and Faller, P. (2006). Structural and thermodynamical properties of CuII amyloid-beta16/28 complexes associated with Alzheimer's disease. J Biol Inorg Chem 11, 1024-38.

[12] Kozin, S.A., Zirah, S., Rebuffat, S., Hoa, G.H. and Debey, P. (2001). Zinc binding to Alzheimer's Abeta(1-16) peptide results in stable soluble complex. Biochem Biophys Res Commun 285, 959-64.

[13] Mekmouche, Y., Coppel, Y., Hochgrafe, K., Guilloreau, L., Talmard, C., Mazarguil, H. and Faller, P. (2005). Characterization of the ZnII binding to the peptide amyloid-beta1-16 linked to Alzheimer's disease. Chembiochem 6, 1663-71.

[14] Zirah, S. et al. (2004). Zinc binding agonist effect on the recognition of the beta-amyloid (4-10) epitope by anti-beta-amyloid antibodies. Biochem Biophys Res Commun 321, 324-8.

[15] Lovell, M.A., Robertson, J.D., Teesdale, W.J., Campbell, J.L. and Markesbery, W.R. (1998). Copper, iron and Zinc in Alzheimer's disease senile plaques. J Neurol Sci 158, 47-52.

[16] Frederickson, C.J. and Bush, A.I. (2001). Synaptically released zinc: physiological functions and pathological effects. Biometals 14, 353-66.

[17] Frederickson, C.J., Suh, S.W., Silva, D. and Thompson, R.B. (2000). Importance of zinc in the central nervous system: the zinc-containing neuron. J Nutr 130, 1471S-83S.

[18] Friedlich, A.L. et al. (2004). Neuronal zinc exchange with the blood vessel wall promotes cerebral amyloid angiopathy in an animal model of Alzheimer's disease. J Neurosci 24, 3453-9.

[19] Bush, A.I. (2003). Copper, zinc, and the metallobiology of Alzheimer disease. Alzheimer Dis Assoc Disord 17, 147-50.

[20] Maynard, C.J., Bush, A.I., Masters, C.L., Cappai, R. and Li, Q.X. (2005). Metals and amyloid-beta in Alzheimer's disease. Int J Exp Pathol 86, 147-59.

[21] Auld, D.S., Kar, S. and Quirion, R. (1998). Beta-amyloid peptides as direct cholinergic neuromodulators: a missing link? Trends Neurosci 21, 43-9.

[22] Kelly, J.F., Furukawa, K., Barger, S.W., Rengen, M.R., Mark, R.J., Blanc, E.M., Roth, G.S. and Mattson, M.P. (1996). Amyloid beta-peptide disrupts carbachol-induced muscarinic cholinergic signal transduction in cortical neurons. Proc Natl Acad Sci U S A 93, 6753-8.

[23] Mijailovic, B., Mladenovic, T., Karadaglic, D., Ninkovic, M., Jovic, P. and Pavlovic, M. (1996). [Clinical and laboratory studies of cholinergic urticaria]. Vojnosanit Pregl 53, 497-501.

[24] Pedersen, W.A., Kloczewiak, M.A. and Blusztajn, J.K. (1996). Amyloid beta-protein reduces acetylcholine synthesis in a cell line derived from cholinergic neurons of the basal forebrain. Proc Natl Acad Sci U S A 93, 8068-71.

[25] Wu, J., Kuo, Y.P., George, A.A., Xu, L., Hu, J. and Lukas, R.J. (2004). beta-Amyloid directly inhibits human alpha4beta2-nicotinic acetylcholine receptors heterologously expressed in human SH-EP1 cells. J Biol Chem 279, 37842-51.

[26] Coyle, J. and Kershaw, P. (2001). Galantamine, a cholinesterase inhibitor that allosterically modulates nicotinic receptors: effects on the course of Alzheimer's disease. Biol Psychiatry 49, 289-99.

[27] Maelicke, A., Samochocki, M., Jostock, R., Fehrenbacher, A., Ludwig, J., Albuquerque, E.X. and Zerlin, M. (2001). Allosteric sensitization of nicotinic receptors by galantamine, a new treatment strategy for Alzheimer's disease. Biol Psychiatry 49, 279-88.

[28] Newhouse, P.A., Potter, A., Kelton, M. and Corwin, J. (2001). Nicotinic treatment of Alzheimer's disease. Biol Psychiatry 49, 268-78.

[29] Wang, H.Y., Li, W., Benedetti, N.J. and Lee, D.H. (2003). Alpha 7 nicotinic acetylcholine receptors mediate beta-amyloid peptide-induced tau protein phosphorylation. J Biol Chem 278, 31547-53.

[30] Christensen, D.D. (2007). Changing the Course of Alzheimer's Disease: Anti-Amyloid Disease-Modifying Treatments on the Horizon. J Clin Psychiatry 9, 32-41.

[31] Gervais, F. et al. (2007). Targeting soluble Abeta peptide with Tramiprosate for the treatment of brain amyloidosis. Neurobiol Aging 28, 537-47.

[32] Velazquez, P., Cribbs, D.H., Poulos, T.L. and Tenner, A.J. (1997). Aspartate residue 7 in amyloid beta-protein is critical for classical complement pathway activation: implications for Alzheimer's disease pathogenesis. Nat Med 3, 77-9.

[33] Giulian, D. et al. (1998). The HHQK domain of beta-amyloid provides a structural basis for the immunopathology of Alzheimer's disease. J Biol Chem 273, 29719-26.

[34] Bronfman, F.C., Garrido, J., Alvarez, A., Morgan, C. and Inestrosa, N.C. (1996). Laminin inhibits amyloid-beta-peptide fibrillation. Neurosci Lett 218, 201-3.

[35] Castillo, G.M., Lukito, W., Peskind, E., Raskind, M., Kirschner, D.A., Yee, A.G. and Snow, A.D. (2000). Laminin inhibition of beta-amyloid protein (Abeta) fibrillogenesis and identification of an Abeta binding site localized to the globular domain repeats on the laminin a chain. J Neurosci Res 62, 451-62.

[36] Murtomaki, S., Risteli, J., Risteli, L., Koivisto, U.M., Johansson, S. and Liesi, P. (1992). Laminin and its neurite outgrowth-promoting domain in the brain in Alzheimer's disease and Down's syndrome patients. J Neurosci Res 32, 261-73.

[37] Caplan, M.R., Schwartzfarb, E.M., Zhang, S., Kamm, R.D. and Lauffenburger, D.A. (2002). Control of self-assembling oligopeptide matrix formation through systematic variation of amino acid sequence. Biomaterials 23, 219-27.

[38] Zhang, S. (2002). Emerging biological materials through molecular self-assembly. Biotechnol Adv 20, 321-39.

[39] Buneeva, O. Proteomics 2010, 10, 23-37.

**Revendications**

1. Composé peptidique de formule générale suivante (I) ou (II):

$$R^a -R^1-R^2-R^3-R^4-R^b \text{ (I) ou } R^a -R^4-R^3-R^2-R^1-R^b \qquad \text{(II)}$$

Dans lesquelles,

   - $R^a$ représente la fonction amine primaire N-terminale de l'acide aminé $R^1$ ou $R^4$, libre ou substituée par un groupe protecteur, et
   - $R^b$ représente la fonction hydroxyle du groupe carboxyle C-terminale de l'acide aminé $R^1$ ou $R^4$, libre ou substituée par un groupe protecteur, et
   - $R^1-R^2-R^3-R^4$ ou $R^4-R^3-R^2-R^1$ représentent HADD, KADD, DDAK, RADD, DDAR, KAED, DEAK, RAED, DEAR, HADE, EDAH, KADE, EDAK, RADE, EDAR, HAEE, EEAH, KAEE, EEAK, RAEE et EEAR, EDAK étant un peptide dans lequel $R^a$ représente une dite fonction amine N-terminale substituée par un groupe protecteur et $R^b$ représente une dite fonction hydroxyle C-terminale substituée par un groupe protecteur.

2. Composé peptidique selon la revendication 1, **caractérisé en ce qu'**il est choisi parmi les composés de formule (I) ou (II) dans lesquelles $R^1-R^2-R^3-R^4$ ou $R^4-R^3-R^2-R^1$ représentent HAEE, EDAR, EEAH, EEAK, EEAR, HADD, HADE, KADD, KAEE, RADD, RADE, RAEE, DDAK, KAED, DEAR ou KADE.

3. Composé peptidique selon la revendication 2, **caractérisé en ce qu'**il est choisi parmi les composés de formule (I) ou (II), dans lesquels $R^1-R^2-R^3-R^4$ ou $R^4-R^3-R^2-R^1$ représentent l'un des tétrapeptides HADD, DDAK, RADD, KAED, DEAR, KADE, HAEE et RAEE.

4. Composé peptidique selon la revendication 2, **caractérisé en ce qu'**il est choisi parmi les composés de formule (I) ou (II), dans lesquels $R^1-R^2-R^3-R^4$ ou $R^4-R^3-R^2-R^1$ représentent l'un des deux tétrapeptides HAEE et RADD.

5. Composé peptidique selon l'une des revendications 1 à 4, **caractérisé en ce que** son degré de pureté est d'au moins 98%, de préférence au moins 99%, et les acides aminés $R^1$, $R^2$, $R^3$ et $R^4$ sont sous forme d'isomères naturels L, et $R^a$ et $R^b$ représentent des dits groupes protecteurs.

6. Composé peptidique selon l'une des revendications 1 à 5, **caractérisé en ce que** $R^a$ représente une dite fonction amine N-terminale substituée par un groupe formyle ou acétyle et $R^b$ représente une dite fonction hydroxyle C-terminale protégée par un groupe -$NH_2$, -NHR, NRR avec R qui est un alkyle en C1 à C4, ou encore -O-R, avec R qui est un alkyle en C1 à C4 ou un alkylamine.

7. Composé peptidique selon l'une des revendications 1 à 6, **caractérisé en ce qu'**il répond à la formule Ac-HAEE-$NH_2$ ou Ac-RADD-$NH_2$.

8. Composé peptidique selon l'une des revendications 1 à 7, pour une utilisation pour réduire ou empêcher la liaison des ions Zn(II) au peptide amyloïde $\beta$, par liaison dudit composé au dit peptide amyloïde $\beta$.

9. Composé peptidique selon l'une des revendications 1 à 7, pour une utilisation pour réduire ou empêcher la polymérisation et/ou agrégation du peptide amyloïde $\beta$ en présence d'ions Zn(II), par liaison dudit composé au dit peptide amyloïde $\beta$.

10. Composé peptidique selon l'une des revendications 1 à 7, pour une utilisation à titre de médicament.

11. Composé peptidique pour une utilisation selon la revendication 10, pour le traitement d'une maladie dans laquelle on constate la formation de plaques amyloïdes.

12. Composé peptidique pour une utilisation selon la revendication 10 ou 11, à titre de médicament pour le traitement d'une maladie neurodégénérative.

13. Composé peptidique pour une utilisation selon la revendication 12, à titre de médicament pour le traitement de la maladie d'Alzheimer.

14. Composition comprenant une substance active comprenant au moins un reste $R^1$-$R^2$-$R^3$-$R^4$ ou $R^4$-$R^3$-$R^2$-$R^1$ d'un composé peptidique selon l'une des revendications 1 à 7.

15. Composition pharmaceutique comprenant une composition selon la revendication 14, avec un véhicule pharmaceutiquement acceptable de préférence pour une administration parentérale, transcutanée ou transmuqueuse ou un véhicule approprié pour une administration intravasculaire, intramusculaire, sous-cutanée intrarachidienne ou cérébrospinale.

**Patentansprüche**

1. Peptidverbindung der folgenden allgemeinen Formel (I) oder (II):

$$R^a\text{-}R^1\text{-}R^2\text{-}R^3\text{-}R^4\text{-}R^b \text{ (I) oder } R^a\text{-}R^4\text{-}R^3\text{-}R^2\text{-}R^1\text{-}R^b \qquad \text{(II)}$$

in welchen

- $R^a$ für die primäre N-terminale Amin-Funktion der Aminosäure $R^1$ oder $R^4$ steht, die frei oder durch eine Schutzgruppe substituiert ist, und
- $R^b$ für die Hydroxyl-Funktion der C-terminalen Carboxyl-Gruppe der Aminosäure $R^1$ oder $R^4$ steht, die frei oder durch eine Schutzgruppe substituiert ist, und
- $R^1$-$R^2$-$R^3$-$R^4$ oder $R^4$-$R^3$-$R^2$-$R^1$ für HADD, KADD, DDAK, RADD, DDAR, KAED, DEAK, RAED, DEAR, HADE, EDAH, KADE, EDAK, RADE, EDAR, HAEE, EEAH, KAEE, EEAK, RAEE und EEAR steht, wobei EDAK ein Peptid ist, in welchem $R^a$ für eine solche N-terminale Amin-Funktion steht, die durch eine Schutzgruppe substituiert ist, und $R^b$ für eine solche C-terminale Hydroxyl-Funktion steht, die durch eine Schutzgruppe substituiert ist.

2. Peptidverbindung nach Anspruch 1, **dadurch gekennzeichnet, dass** sie aus den Verbindungen der Formel (I) oder (II) ausgewählt ist, in welchen $R^1$-$R^2$-$R^3$-$R^4$ oder $R^4$-$R^3$-$R^2$-$R^1$ für HAEE, EDAR, EEAH, EEAK, EEAR, HADD, HADE, KADD, KAEE, RADD, RADE, RAEE, DDAK, KAED, DEAR oder KADE steht.

3. Peptidverbindung nach Anspruch 2, **dadurch gekennzeichnet, dass** sie aus den Verbindungen der Formel (I) oder (II) ausgewählt ist, in welchen $R^1$-$R^2$-$R^3$-$R^4$ oder $R^4$-$R^3$-$R^2$-$R^1$ für eines der Tetrapeptide HADD, DDAK, RADD, KAED, DEAR, KADE, HAEE und RAEE steht.

4. Peptidverbindung nach Anspruch 2, **dadurch gekennzeichnet, dass** sie aus den Verbindungen der Formel (I) oder (II) ausgewählt ist, in welchen $R^1$-$R^2$-$R^3$-$R^4$ oder $R^4$-$R^3$-$R^2$-$R^1$ für eines der zwei Tetrapeptide HAEE und RADD steht.

5. Peptidverbindung nach einem der Ansprüche 1 bis 4, **dadurch gekennzeichnet, dass** ihr Reinheitsgrad mindestens 98 %, vorzugsweise mindestens 99 %, beträgt, und die Aminosäuren $R^1$, $R^2$, $R^3$ und $R^4$ in Form von natürlichen Isomeren L vorliegen, und $R^a$ und $R^b$ für die Schutzgruppen stehen.

6. Peptidverbindung nach einem der Ansprüche 1 bis 5, **dadurch gekennzeichnet, dass** $R^a$ für eine solche N-terminale Amin-Funktion steht, die durch eine Formyl- oder Acetyl-Gruppe substituiert ist, und $R^b$ für eine solche C-terminale Hydroxyl-Funktion steht, die durch eine Gruppe -$NH_2$, -NHR, NRR geschützt ist, wobei R ein C1- bis C4-Alkyl ist, oder auch -O-R, wobei R ein C1- bis C4-Alkyl oder ein Alkylamin ist.

**7.** Peptidverbindung nach einem der Ansprüche 1 bis 6, **dadurch gekennzeichnet, dass** sie der Formel Ac-HAEE-$NH_2$ oder Ac-RADD-$NH_2$ entspricht.

**8.** Peptidverbindung nach einem der Ansprüche 1 bis 7, für eine Verwendung zur Reduktion oder Verhinderung der Bindung von Zn(II)-Ionen an das Peptid β-Amyloid durch Bindung der Verbindung an das Peptid β-Amyloid.

**9.** Peptidverbindung nach einem der Ansprüche 1 bis 7, für eine Verwendung zur Reduktion oder Verhinderung der Polymerisation und/oder Aggregation des Peptids β-Amyloid in Anwesenheit von Zn(II)-Ionen durch Bindung der Verbindung an das Peptid β-Amyloid.

**10.** Peptidverbindung nach einem der Ansprüche 1 bis 7, für eine Verwendung als Medikament.

**11.** Peptidverbindung für eine Verwendung nach Anspruch 10, zur Behandlung einer Erkrankung, bei welcher die Bildung von Amyloidplaques festgestellt wird.

**12.** Peptidverbindung für eine Verwendung nach Anspruch 10 oder 11, als Medikament für die Behandlung einer neurodegenerativen Erkrankung.

**13.** Peptidverbindung für eine Verwendung nach Anspruch 12, als Medikament für die Behandlung von Morbus Alzheimer.

**14.** Zusammensetzung, die eine aktive Substanz umfasst, die zumindest einen Rest $R^1$-$R^2$-$R^3$-$R^4$ oder $R^4$-$R^3$-$R^2$-$R^1$ einer Peptidverbindung nach einem der Ansprüche 1 bis 7 umfasst.

**15.** Pharmazeutische Zusammensetzung, die eine Zusammensetzung nach Anspruch 14 umfasst, mit einem pharmazeutisch akzeptablen Träger, vorzugsweise für eine parenterale, transkutane oder transmukosale Verabreichung, oder einem Träger, der für eine intravaskuläre, intramuskuläre, subkutane intraspinale oder zerebrospinale Verabreichung geeignet ist.

**Claims**

**1.** Peptide compound of the following general formula (I) or (II):

$$R^a\text{-}R^1\text{-}R^2\text{-}R^3\text{-}R^4\text{-}R^b \text{ (I) or } R^a\text{-}R^4\text{-}R^3\text{-}R^2\text{-}R^1\text{-}R^b \qquad \text{(II)}$$

Wherein,

- $R^a$ represents the N-terminal primary amine function of the amino acid $R^1$ or $R^4$, free or substituted by a protecting group, and
- $R^b$ represents the hydroxyl function of the C-terminal carboxyl group of the amino acid $R^1$ or $R^4$, free or substituted by a protecting group, and
- $R^1$-$R^2$-$R^3$-$R^4$ or $R^4$-$R^3$-$R^2$-$R^1$ represent HADD, KADD, DDAK, RADD, DDAR, KAED, DEAK, RAED, DEAR, HADE, EDAH, KADE, EDAK, RADE, EDAR, HAEE, EEAH, KAEE, EEAK, RAEE and EEAR, EDAK being a peptide wherein $R^a$ represents a said N-terminal amine function substituted by a protecting group and $R^b$ represents a said C-terminal hydroxyl function substituted by a protecting group.

**2.** Peptide compound according to claim 1, **characterized in that** it is selected from compounds of formula (I) or (II) wherein $R^1$-$R^2$-$R^3$-$R^4$ or $R^4$-$R^3$-$R^2$-$R^1$ represent HAEE, EDAR, EEAH, EEAK, EEAR, HADD, HADE, KADD, KAEE, RADD, RADE, RAEE, DDAK, KAED, DEAR or KADE.

**3.** Peptide compound according to claim 2, **characterized in that** it is selected from compounds of formula (I) or (II), wherein $R^1$-$R^2$-$R^3$-$R^4$ or $R^4$-$R^3$-$R^2$-$R^1$ represent one of the tetrapeptides HADD, DDAK, RADD, KAED, DEAR, KADE, HAEE and RAEE.

**4.** Peptide compound according to claim 2, **characterized in that** it is selected from compounds of formula (I) or (II), wherein $R^1$-$R^2$-$R^3$-$R^4$ or $R^4$-$R^3$-$R^2$-$R^1$ represent one of the two tetrapeptides HAEE and RADD.

5. Peptide compound according to one of claims 1 to 4, **characterized in that** its purity is at least 98%, preferably at least 99%, and the amino acids $R^1$, $R^2$, $R^3$ and $R^4$ are in the form of natural L isomers, and $R^a$ and $R^b$ represent said protecting groups.

6. Peptide compound according to one of claims 1 to 5, **characterized in that** $R^a$ represents a said N-terminal amine function substituted by a formyl or acetyl group and $R^b$ represents a said C-terminal hydroxyl function protected by an -$NH_2$, -NHR, NRR group with R which is a C1 to C4 alkyl, or -O-R, with R which is a C1 to C4 alkyl or an alkylamine.

7. Peptide compound according to one of claims 1 to 6, **characterized in that** it has the formula Ac-HAEE-$NH_2$ or Ac-RADD-$NH_2$.

8. Peptide compound according to one of claims 1 to 7, for use in reducing or preventing the binding of Zn(II) ions to the amyloid β-peptide, by binding said compound to said amyloid β-peptide.

9. Peptide compound according to one of claims 1 to 7, for use in reducing or preventing polymerization and/or aggregation of the amyloid β-peptide in the presence of Zn(II) ions, by binding said compound to said amyloid β-peptide.

10. Peptide compound according to one of claims 1 to 7, for use as a medicinal product.

11. Peptide compound for use according to claim 10, for the treatment of a disease in which amyloid plaques are formed.

12. Peptide compound for use according to claim 10 or 11, as a medicinal product for the treatment of neurodegenerative disease.

13. Peptide compound for use according to claim 12 as a medicinal product for the treatment of Alzheimer's disease.

14. Composition comprising an active substance comprising at least one residue $R^1$-$R^2$-$R^3$-$R^4$ or $R^4$-$R^3$-$R^2$-$R^1$ of a peptide compound according to one of claims 1 to 7.

15. Pharmaceutical composition comprising a composition according to claim 14, with a pharmaceutically acceptable carrier preferably for parenteral, transcutaneous or transmucosal administration or a carrier suitable for intravascular, intramuscular, subcutaneous, intrathecal or cerebrospinal administration.

FIG.1

FIG.2

FIG.3

FIG.4

FIG.5

FIG.6

FIG.7

FIG.8

FIG.9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6001852 A
- US 7018797 B
- US 7314724 B
- WO 9313789 A

- US 2004214165 A
- EP 2130550 A
- FR 1058827

**Littérature non-brevet citée dans la description**

- **GOMEZ-RUIZ et al.** *J.Dairy Sci.,* 2007, vol. 90, 4966-4973
- **CHU et al.** *J.Am. Chem. Soc.,* 1995, vol. 117, 5419-54205
- **CUMMINGS, J.L.** Alzheimer's disease. *N Engl J Med,* 2004, vol. 351, 56-67
- **SELKOE, D.J.** Alzheimer's disease: genes, proteins, and therapy. *Physiol Rev,* 2001, vol. 81, 741-66
- **GLENNER, G.G. ; WONG, C.W.** Alzheimer's disease and Down's syndrome: sharing of a unique cerebrovascular amyloid fibril protein. *Biochem Biophys Res Commun,* 1984, vol. 122, 1131-5
- **MASTERS, C.L. ; SIMMS, G. ; WEINMAN, N.A. ; MULTHAUP, G. ; MCDONALD, B.L. ; BEYREUTHER, K.** Amyloid plaque core protein in Alzheimer disease and Down syndrome. *Proc Natl Acad Sci U S A,* 1985, vol. 82, 4245-9
- **MAYEUX, R. et al.** Plasma amyloid beta-peptide 1-42 and incipient Alzheimer's disease. *Ann Neurol,* 1999, vol. 46, 412-6
- **SEUBERT, P. et al.** Isolation and quantification of soluble Alzheimer's beta-peptide from biological fluids. *Nature,* 1992, vol. 359, 325-7
- **HARDY, J. ; SELKOE, D.J.** The amyloid hypothesis of Alzheimer's disease: progress and problems on the road to therapeutics. *Science,* 2002, vol. 297, 353-6
- **LUHRS, T. ; RITTER, C. ; ADRIAN, M. ; RIEK-LOHER, D. ; BOHRMANN, B. ; DOBELI, H. ; SCHUBERT, D. ; RIEK, R.** 3D structure of Alzheimer's amyloid-beta(1-42) fibrils. *Proc Natl Acad Sci U S A,* 2005, vol. 102, 17342-7
- **GOWING, E. ; ROHER, A.E. ; WOODS, A.S. ; COTTER, R.J. ; CHANEY, M. ; LITTLE, S.P. ; BALL, M.J.** Chemical characterization of A beta 17-42 peptide, a component of diffuse amyloid deposits of Alzheimer disease. *J Biol Chem,* 1994, vol. 269, 10987-90

- **MATTSON, M.P.** Untangling the pathophysiochemistry of beta-amyloid. *Nat Struct Biol,* 1995, vol. 2, 926-8
- **GUILLOREAU, L. ; DAMIAN, L. ; COPPEL, Y. ; MAZARGUIL, H. ; WINTERHALTER, M. ; FALLER, P.** Structural and thermodynamical properties of CuII amyloid-beta16/28 complexes associated with Alzheimer's disease. *J Biol Inorg Chem,* 2006, vol. 11, 1024-38
- **KOZIN, S.A. ; ZIRAH, S. ; REBUFFAT, S. ; HOA, G.H. ; DEBEY, P.** Zinc binding to Alzheimer's Abeta(1-16) peptide results in stable soluble complex. *Biochem Biophys Res Commun,* 2001, vol. 285, 959-64
- **MEKMOUCHE, Y. ; COPPEL, Y. ; HOCHGRAFE, K. ; GUILLOREAU, L. ; TALMARD, C. ; MAZARGUIL, H. ; FALLER, P.** Characterization of the ZnII binding to the peptide amyloid-beta1-16 linked to Alzheimer's disease. *Chembiochem,* 2005, vol. 6, 1663-71
- **ZIRAH, S. et al.** Zinc binding agonist effect on the recognition of the beta-amyloid (4-10) epitope by anti-beta-amyloid antibodies. *Biochem Biophys Res Commun,* 2004, vol. 321, 324-8
- **LOVELL, M.A. ; ROBERTSON, J.D. ; TEESDALE, W.J. ; CAMPBELL, J.L. ; MARKESBERY, W.R.** Copper, iron and Zinc in Alzheimer's disease senile plaques. *J Neurol Sci,* 1998, vol. 158, 47-52
- **FREDERICKSON, C.J. ; BUSH, A.I.** Synaptically released zinc: physiological functions and pathological effects. *Biometals,* 2001, vol. 14, 353-66
- **FREDERICKSON, C.J. ; SUH, S.W. ; SILVA, D. ; THOMPSON, R.B.** Importance of zinc in the central nervous system: the zinc-containing neuron. *J Nutr,* 2000, vol. 130, 1471S-83S
- **FRIEDLICH, A.L. et al.** Neuronal zinc exchange with the blood vessel wall promotes cerebral amyloid angiopathy in an animal model of Alzheimer's disease. *J Neurosci,* 2004, vol. 24, 3453-9

- **BUSH, A.I.** Copper, zinc, and the metallobiology of Alzheimer disease. *Alzheimer Dis Assoc Disord,* 2003, vol. 17, 147-50
- **MAYNARD, C.J. ; BUSH, A.I. ; MASTERS, C.L. ; CAPPAI, R. ; LI, Q.X.** Metals and amyloid-beta in Alzheimer's disease. *Int J Exp Pathol,* 2005, vol. 86, 147-59
- **AULD, D.S. ; KAR, S. ; QUIRION, R.** Beta-amyloid peptides as direct cholinergic neuromodulators: a missing link?. *Trends Neurosci,* 1998, vol. 21, 43-9
- **KELLY, J.F. ; FURUKAWA, K. ; BARGER, S.W. ; RENGEN, M.R. ; MARK, R.J. ; BLANC, E.M. ; ROTH, G.S. ; MATTSON, M.P.** Amyloid beta-peptide disrupts carbachol-induced muscarinic cholinergic signal transduction in cortical neurons. *Proc Natl Acad Sci U S A,* 1996, vol. 93, 6753-8
- **MIJAILOVIC, B. ; MLADENOVIC, T. ; KARA-DAGLIC, D. ; NINKOVIC, M. ; JOVIC, P. ; PAV-LOVIC, M.** Clinical and laboratory studies of cholinergic urticaria. *Vojnosanit Pregl,* 1996, vol. 53, 497-501
- **PEDERSEN, W.A. ; KLOCZEWIAK, M.A. ; BLUSZ-TAJN, J.K.** Amyloid beta-protein reduces acetylcholine synthesis in a cell line derived from cholinergic neurons of the basal forebrain. *Proc Natl Acad Sci U S A,* 1996, vol. 93, 8068-71
- **WU, J. ; KUO, Y.P. ; GEORGE, A.A. ; XU, L. ; HU, J. ; LUKAS, R.J.** beta-Amyloid directly inhibits human alpha4beta2-nicotinic acetylcholine receptors heterologously expressed in human SH-EP1 cells. *J Biol Chem,* 2004, vol. 279, 37842-51
- **COYLE, J. ; KERSHAW, P.** Galantamine, a cholinesterase inhibitor that allosterically modulates nicotinic receptors: effects on the course of Alzheimer's disease. *Biol Psychiatry,* 2001, vol. 49, 289-99
- **MAELICKE, A. ; SAMOCHOCKI, M. ; JOSTOCK, R. ; FEHRENBACHER, A. ; LUDWIG, J. ; ALBU-QUERQUE, E.X. ; ZERLIN, M.** Allosteric sensitization of nicotinic receptors by galantamine, a new treatment strategy for Alzheimer's disease. *Biol Psychiatry,* 2001, vol. 49, 279-88
- **NEWHOUSE, P.A. ; POTTER, A. ; KELTON, M. ; CORWIN, J.** Nicotinic treatment of Alzheimer's disease. *Biol Psychiatry,* 2001, vol. 49, 268-78
- **WANG, H.Y. ; LI, W. ; BENEDETTI, N.J. ; LEE, D.H.** Alpha 7 nicotinic acetylcholine receptors mediate beta-amyloid peptide-induced tau protein phosphorylation. *J Biol Chem,* 2003, vol. 278, 31547-53
- **CHRISTENSEN, D.D.** Changing the Course of Alzheimer's Disease: Anti-Amyloid Disease-Modifying Treatments on the Horizon. *J Clin Psychiatry,* 2007, vol. 9, 32-41
- **GERVAIS, F. et al.** Targeting soluble Abeta peptide with Tramiprosate for the treatment of brain amyloidosis. *Neurobiol Aging,* 2007, vol. 28, 537-47
- **VELAZQUEZ, P. ; CRIBBS, D.H. ; POULOS, T.L. ; TENNER, A.J.** Aspartate residue 7 in amyloid beta-protein is critical for classical complement pathway activation: implications for Alzheimer's disease pathogenesis. *Nat Med,* 1997, vol. 3, 77-9
- **GIULIAN, D. et al.** The HHQK domain of beta-amyloid provides a structural basis for the immunopathology of Alzheimer's disease. *J Biol Chem,* 1998, vol. 273, 29719-26
- **BRONFMAN, F.C. ; GARRIDO, J. ; ALVAREZ, A. ; MORGAN, C. ; INESTROSA, N.C.** Laminin inhibits amyloid-beta-peptide fibrillation. *Neurosci Lett,* 1996, vol. 218, 201-3
- **CASTILLO, G.M. ; LUKITO, W. ; PESKIND, E. ; RASKIND, M. ; KIRSCHNER, D.A. ; YEE, A.G. ; SNOW, A.D.** Laminin inhibition of beta-amyloid protein (Abeta) fibrillogenesis and identification of an Abeta binding site localized to the globular domain repeats on the laminin a chain. *J Neurosci Res,* 2000, vol. 62, 451-62
- **MURTOMAKI, S. ; RISTELI, J. ; RISTELI, L. ; KOIVISTO, U.M. ; JOHANSSON, S. ; LIESI, P.** Laminin and its neurite outgrowth-promoting domain in the brain in Alzheimer's disease and Down's syndrome patients. *J Neurosci Res,* 1992, vol. 32, 261-73
- **CAPLAN, M.R. ; SCHWARTZFARB, E.M. ; ZHANG, S. ; KAMM, R.D. ; LAUFFENBURGER, D.A.** Control of self-assembling oligopeptide matrix formation through systematic variation of amino acid sequence. *Biomaterials,* 2002, vol. 23, 219-27
- **ZHANG, S.** Emerging biological materials through molecular self-assembly. *Biotechnol Adv,* 2002, vol. 20, 321-39
- **BUNEEVA, O.** *Proteomics,* 2010, vol. 10, 23-37